(19) Europäisches Patentamt
European Patent Office
Office européen des brevets

(11) EP 4 283 743 A1

(12) EUROPEAN PATENT APPLICATION
published in accordance with Art. 153(4) EPC

(43) Date of publication:
**29.11.2023 Bulletin 2023/48**

(21) Application number: **22742895.0**

(22) Date of filing: **21.01.2022**

(51) International Patent Classification (IPC):
***H01M 10/0567*** (2010.01) ***H01M 10/052*** (2010.01)

(52) Cooperative Patent Classification (CPC):
**H01M 10/052; H01M 10/0567**

(86) International application number:
**PCT/KR2022/001172**

(87) International publication number:
**WO 2022/158919 (28.07.2022 Gazette 2022/30)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.01.2021 KR 20210009629**
**20.10.2021 KR 20210140439**

(71) Applicant: **Soulbrain Co., Ltd.**
**Seongnam-si, Gyeonggi-do 13486 (KR)**

(72) Inventors:
- **JANG, Min Jung**
  **Seongnam-si, Gyeonggi-do 13486 (KR)**
- **KIM, Min Goo**
  **Seongnam-si, Gyeonggi-do 13486 (KR)**
- **LIM, Young Rok**
  **Seongnam-si, Gyeonggi-do 13486 (KR)**
- **CHOI, Ji Young**
  **Seongnam-si, Gyeonggi-do 13486 (KR)**
- **LEE, Sang Ho**
  **Seongnam-si, Gyeonggi-do 13486 (KR)**
- **KANG, Wan Chul**
  **Seongnam-si, Gyeonggi-do 13486 (KR)**
- **YUN, Jong Cheol**
  **Seongnam-si, Gyeonggi-do 13486 (KR)**
- **HAN, Ji Seong**
  **Seongnam-si, Gyeonggi-do 13486 (KR)**
- **RYU, Hee Jeong**
  **Seongnam-si, Gyeonggi-do 13486 (KR)**
- **CHUNG, Jae Won**
  **Seongnam-si, Gyeonggi-do 13486 (KR)**

(74) Representative: **Dehns**
**St. Bride's House**
**10 Salisbury Square**
**London EC4Y 8JD (GB)**

# (54) ELECTROLYTE AND SECONDARY BATTERY COMPRISING SAME

(57) The present invention relates to an electrolyte solution and a secondary battery including the same. According to the present invention, the present invention has an effect of providing a secondary battery having improved charging efficiency and output due to low discharge resistance and having a long lifespan and excellent high-temperature capacity retention by suppressing gas generation and increase in thickness.

EP 4 283 743 A1

## Description

[Technical Field]

The present invention relates to an electrolyte solution and a secondary battery including the same. More particularly, the present invention relates to an electrolyte solution for batteries including electrolyte solution additives capable of improving battery output characteristics and high-temperature storage characteristics and significantly reducing gas generation and a thickness increase rate and a secondary battery including the electrolyte solution.

[Background Art]

In a lithium secondary battery, an electrolyte solution between a cathode and an anode enables smooth movement of lithium ions, and electricity is generated or used by oxidation-reduction reaction according to intercalation and desorption at the cathode and the anode.

A lithium secondary battery is mainly used as a power source for mobile IT devices, such as mobile phones, and electrically-driven tools. With the development of high-capacity technology, the use of lithium secondary batteries is expanding. For example, lithium secondary batteries are used in automobiles and energy storage.

As the application field of secondary batteries expands and the demand for secondary batteries increases, a secondary battery having better performance and stability than those required for conventional small batteries is required. In recent years, various studies on organic solvents or additives as components of electrolyte solutions are being conducted to improve battery characteristics such as output characteristics, cycle characteristics, storage characteristics, and film characteristics.

In the case of a conventional electrolyte solution that does not contain an electrolyte solution additive or contains an electrolyte solution additive with poor properties, it is difficult to expect improvement in low-temperature output characteristics due to the formation of a non-uniform SEI film. In addition, even when an electrolyte solution additive is included, when the input amount thereof is not properly adjusted, due to the electrolyte solution additive, the surface of a cathode is decomposed or an electrolyte solution is oxidized during high temperature reaction, ultimately degrading the cycle characteristics and storage stability of a secondary battery.

[Related Art Documents]

[Patent Documents]

KR 1295395 B1

[Disclosure]

[Technical Problem]

Therefore, the present invention has been made in view of the above problems, and it is one object of the present invention to provide an electrolyte solution for batteries including electrolyte solution additives capable of improving battery output characteristics and high-temperature storage characteristics and significantly reducing gas generation and a thickness increase rate.

It is another object of the present invention to provide an excellent secondary battery having improved battery output due to reduced discharge resistance, being capable of long-term storage due to improved recovery capacity at high temperatures, and being capable of suppressing gas generation in the battery.

The above and other objects can be accomplished by the present invention described below.

[Technical Solution]

In accordance with one aspect of the present invention, provided is an electrolyte solution including an organic solvent, a lithium salt, a first additive, and a second additive,

wherein the first additive includes a compound represented by Formula 1 below in an amount of 20 % by weight or less based on 100 % by weight of the electrolyte solution, and

the second additive consists of 3 to 5 atoms, has 2 to 4 atoms having an electronegativity of 3 or more, has at least one double bond, and includes a compound having an atomic group represented by Formula 2 below in an amount of 0.01 to 10 % by weight based on 100 % by weight of the electrolyte solution.

[Formula 1]

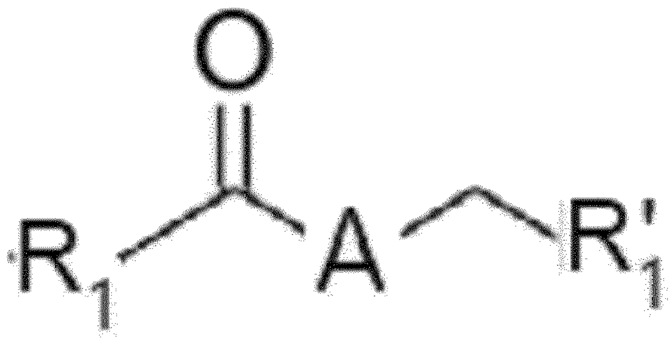

,

wherein A is carbon (C) or oxygen (O); and $R_1$ and $R_1$' are independently a bond or a substituted or unsubstituted alkyl having 1 to 10 carbon atoms and include one or more carbon atoms.

[Formula 2]

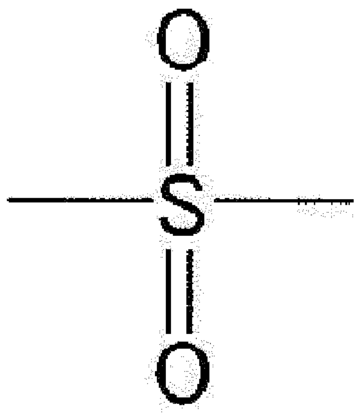

,

wherein a solid line is a bond.

In Formula 1, A may be oxygen (O).

The first additive may be a compound represented by Formula 3 or 4 below.

[Formula 3]

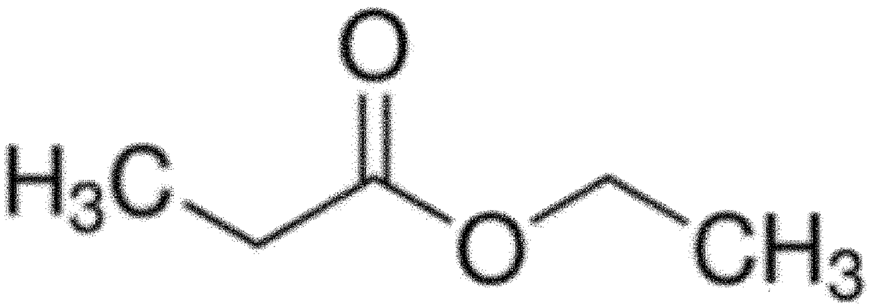

[Formula 4]

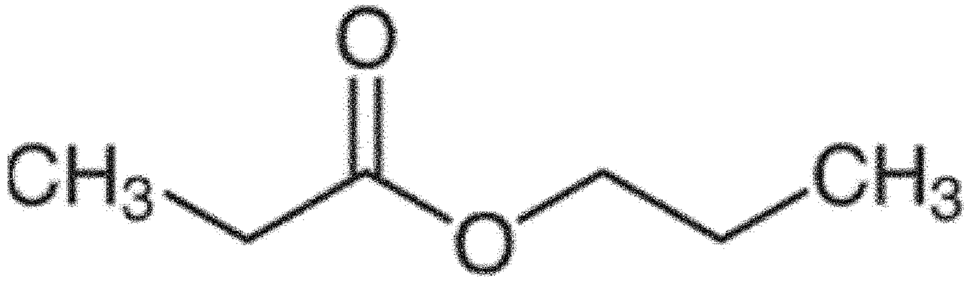

wherein, in Formulas 3 and 4, a solid line is a bond; carbon is located at a point where no separate element is shown and bonds meet; and the number of hydrogens satisfying a valence of the carbon is omitted.

The second additive may have an atomic group represented by Formula 5 below.

[Formula 5]

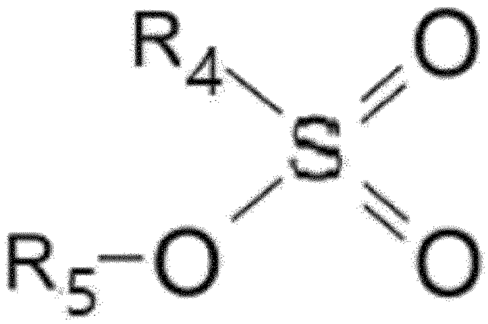

,

wherein $R_4$ is $OR_6$ or $R_6$; $R_5$ is $R_7A$; A is

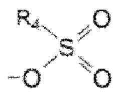

;

and $R_6$ and $R_7$ are independently an alkyl group having 1 to 5 carbon atoms or an aryl group having 6 to 10 carbon atoms.

The second additive may be one or more compound represented by Formula 6 below.

[Formula 6]

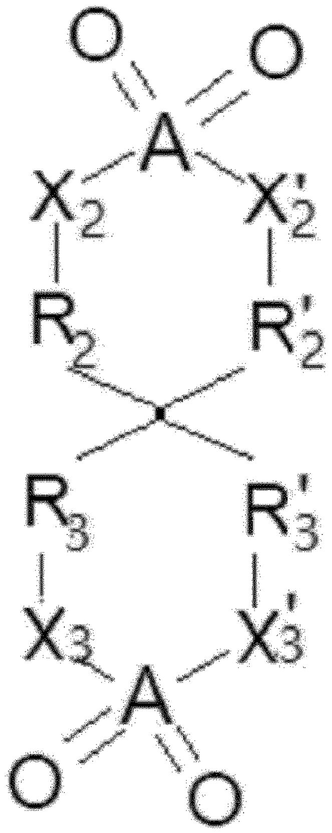

,

wherein A is phosphorus (P), sulfur (S), or nitrogen (N); $X_1$, $X_2$, $X_3$, $X_1$', $X_2$', and $X_3$' are independently a bond or oxygen; n is an integer from 0 to 3; and $R_2$, $R_2$, $R_3$, $R_1$', $R_2$', and $R_3$' are independently a bond or a substituted or unsubstituted alkylene having 1 to 10 carbon atoms and include one or more carbon atoms.

The second additive may be a compound represented by Formula 7 below.

[Formula 7]

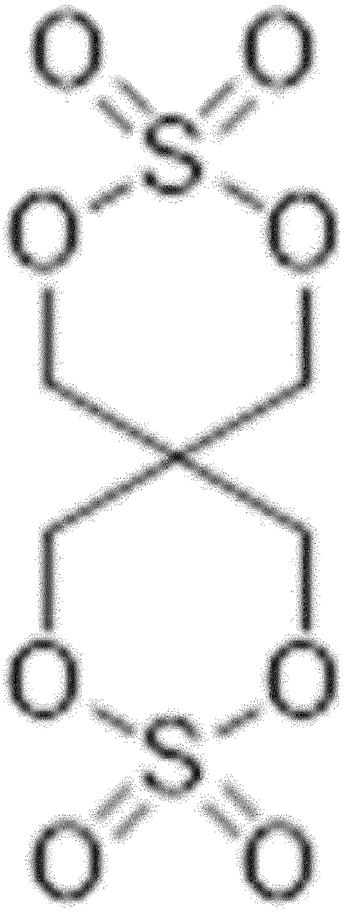

,

wherein a solid line is a bond; carbon is located at a point where no separate element is shown and bonds meet; and the number of hydrogens satisfying a valence of the carbon is omitted.

The first and second additives may be included in a weight ratio of 1:0.1 to 1.7 (first additive:second additive).

The lithium salt may include one or more selected from the group consisting of $LiPF_6$, $LiClO_4$, LiTFSI, $LiAsF_6$, $LiBF_4$, $LiBF_6$, $LiSbF_6$, $LiAl0_4$, $LiAlCl_4$, LiBOB, $LiB_{10}Cl_{10}$, $LiAlCl_4$, $LiAlO_4$, $LiClO_4$, $LiCF_3SO_3$, $LiCH_3CO_2$, $LiCF_3CO_2$, $LiAsF_6$, $LiSbF_6$, $LiCH_3SO_3$, LiBeTI, $LiC_4F_9SO_3$, $LiN(C_2F_5SO_3)_2$, $LiN(C_2F_5SO_2)_2$, $LiN(CF_3SO_2)_2$, $LiN(CaF_{2a+1}SO_2)(C_bF_{2b+1}SO_2)$ (a and b are natural numbers), LiCl, LiI, LiBr, and $LiB(C_2O_4)_2$.

The organic solvent may include two or more selected from the group consisting of ethylene carbonate (EC), propylene carbonate (PC), butylene carbonate (BC), vinylene carbonate (VC), dimethyl carbonate (DMC), diethyl carbonate (DEC), dipropyl carbonate (DPC), ethyl methyl carbonate (EMC), methyl ethyl carbonate (MEC), fluoroethylene carbonate (FEC), methyl propyl carbonate (MPC), ethyl propyl carbonate (EPC), 1,2-butylene carbonate, 2,3-butylene carbonate, 1,2-pentylene carbonate, 2,3-butylene carbonate, methyl acetate, ethyl acetate, propyl acetate, methyl propionate, butyl propionate, γ-butyrolactone, γ-valerolactone, γ-caprolactone, σ-valerolactone, and ε-caprolactone.

Based on 100 % by weight in total of the electrolyte solution, the electrolyte solution may include one or more third additives selected from the group consisting of boron compounds, phosphorus compounds, sulfur compounds, and nitrogenous compounds in an amount of 10 % by weight or less.

In accordance with another aspect of the present invention, provided are electrolyte solution additives including:

a compound represented by Formula 1 below; and
a compound represented by Formula 4 below.

[Formula 1]

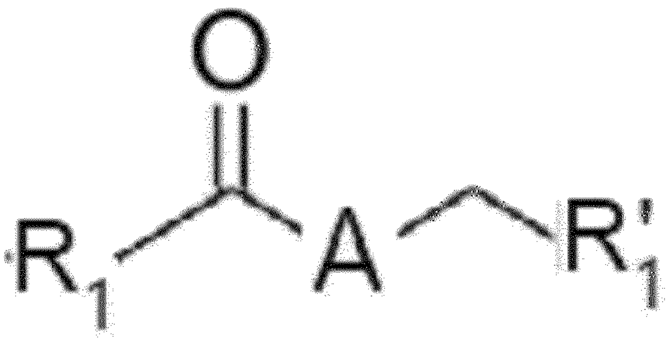

,

wherein A is carbon (C) or oxygen (O); and $R_1$ and $R_1$' are independently a bond or a substituted or unsubstituted alkyl having 1 to 10 carbon atoms and include one or more carbon atoms.

[Formula 5]

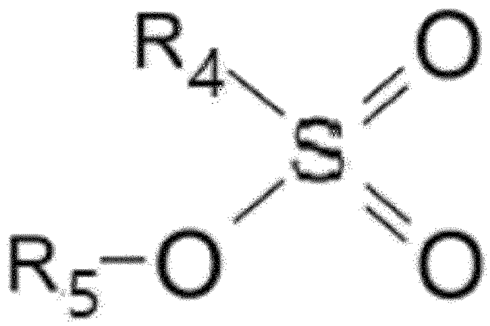

,

wherein $R_4$ is $OR_6$ or $R_6$; $R_5$ is $R_7A$; A is

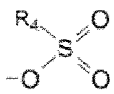

;

and $R_6$ and $R_7$ are independently an alkyl group having 1 to 5 carbon atoms or an aryl group having 6 to 10 carbon atoms.

The compound represented by Formula 1 and the compound represented by Formula 5 may be included in a weight ratio of 1:0.1 to 1.7 (first additive:second additive).

In accordance with yet another aspect of the present invention, provided is a lithium secondary battery including:

an anode, a cathode, a separator disposed between the anode and the cathode, and an electrolyte solution, wherein the electrolyte solution is the above-described electrolyte solution.

The lithium secondary battery may have a discharge resistance value of 40 mΩ or less at 60 °C.

The lithium secondary battery may have a recovery capacity of 1,000 mAh or more at 60 °C.

The lithium secondary battery may have a thickness increase rate of 10.81 % or less as measured at 60 °C and calculated by Equation 1 below.

[Equation 1]

Thickness increase rate (%) = {(Thickness after storage at high temperature - Initial thickness) / Initial thickness} × 100

The lithium secondary battery may have a coulomb efficiency of 99.4 % or more as calculated by Equation 2 below after 300 cycles.

[Equation 2]

Coulomb efficiency (%) = (Discharge capacity at 300 cycles / charge capacity at 300 cycles) × 100

The lithium secondary battery may be a battery for energy storage systems (ESSs) or a battery for automobiles.

[Advantageous Effects]

When an electrolyte solution for batteries according to the present invention is included in a secondary battery, due to decrease in discharge resistance, the output of the secondary battery can be improved. In addition, the secondary battery can have a long lifespan and excellent high-temperature capacity retention due to improved recovery capacity at high temperatures.

In particular, the additive for batteries according to the present invention can suppress gas generation and thickness increase in a battery, thereby providing a secondary battery having excellent performance and lifespan characteristics.

[Best Mode]

Hereinafter, electrolyte solution additives, an electrolyte solution for batteries, and a secondary battery including the electrolyte solution according to the present invention will be described in detail.

The present inventors have studied to develop a secondary battery for automobiles having improved output and having excellent recovery capacity and lifespan characteristics at high temperatures. While conducting the above research, the present inventors confirmed that all of the above objects were achieved when an additive having a specific structure was added to an electrolyte solution of a secondary battery. Based on these results, the present inventors conducted further studies to complete the present invention.

The electrolyte solution additives of the present invention include a first additive and a second additive. The first additive is a compound represented by Formula 1 below. The second additive consists of 3 to 5 atoms, has 2 to 4 atoms having an electronegativity of 3 or more, has at least one double bond, and includes a compound having an atomic group represented by Formula 2 below. In this case, battery output may be improved due to reduced discharge resistance, long-term storage may be realized due to improved recovery capacity at high temperatures, and lifespan maintenance at high temperatures may be excellent.

[Formula 1]

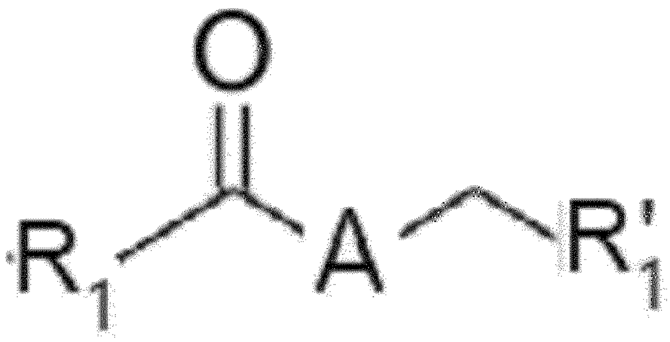

In Formula 1, A is carbon (C) or oxygen (O); and $R_1$ and $R_1$' are independently a bond or a substituted or unsubstituted alkyl having 1 to 10 carbon atoms and include one or more carbon atoms.

[Formula 2]

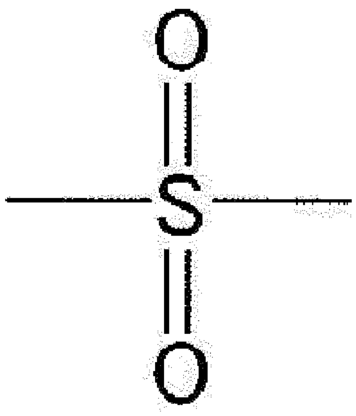

In Formula 2, a solid line is a bond.

In addition, the electrolyte solution for batteries of the present invention includes the electrolyte solution additives.

When the compound represented by Formula 1 is added to the electrolyte solution of a secondary battery, electrons are localized toward O element due to the electronegativity difference between C element and O element directly bonded thereto. Accordingly, C element becomes electron-poor ($e^-$ poor, $\delta$+) state, and oxidation reaction is induced in the electrolyte solution containing lithium ions, thereby forming a stable film on an electrode, specifically, a cathode. At this time, decomposition of the electrolyte solution may be prevented due to the stability of the film, and thus cycle characteristics may be improved. In particular, compared to conventional electrode films that decompose at high temperatures and have poor high-temperature storage properties, the film does not decompose at high temperatures and thus has excellent high-temperature storage properties. In addition, resistance increase may be prevented, and thus charging efficiency and output may be improved. In addition, the safety of a battery may be improved because gas generation due to chemical reaction inside the battery is suppressed. In addition, structural collapse of the electrode active materials of a cathode and anode may be prevented at high temperatures, thereby improving capacity retention. As a result, the effect of increasing a lifespan may be obtained.

As a specific example, in Formula 1, A may be oxygen (O). The first additive may be a compound represented by Formula 3 or 4.

When a lithium secondary battery is manufactured using the electrolyte solution including the first additive, decomposition of the electrolyte solution may be suppressed by the terminal group of the compound, thereby reducing an internal resistance increase rate, gas generation, and a thickness increase rate. As a result, a battery lifespan may be increased.

[Formula 3]

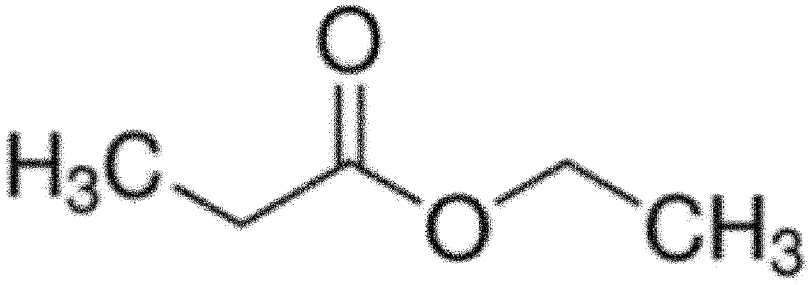

[Formula 4]

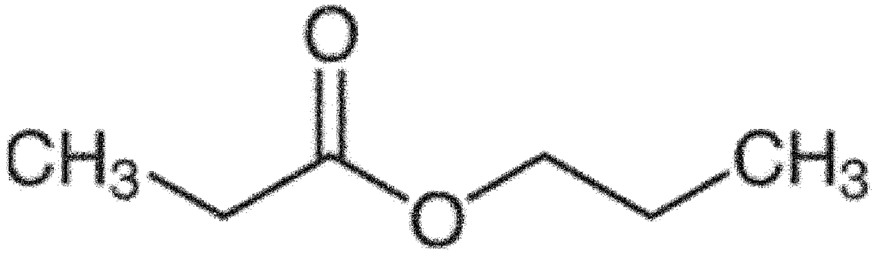

In Formulas 3 and 4, a solid line is a bond; carbon is located at a point where no separate element is shown and bonds meet; and the number of hydrogens satisfying a valence of the carbon is omitted.

As a specific example, when the first additive has the structure represented by Formula 1, since the discharge resistance of a secondary battery including the additive is reduced, the output of the battery may be improved, charging recovery capacity at high temperatures may be increased, and lifespan efficiency may be increased. Thus, the additive is preferably used as an electrolyte solution additive for batteries.

Preferably, $R_1$ and $R_2$ are each independently a bond or a substituted or unsubstituted alkylene having 1 to 10 carbon atoms and include one or more carbon atoms. When both $R_1$ and $R_2$ are an unsubstituted alkylene having 1 to 10 carbon atoms, the compound is chemically stabilized and inactivated, and the molecular structure thereof is simplified and stabilized.

In particular, when an asymmetrical alkyl group is included, flow of electrons in a molecule is stabilized, thereby increasing molecular rigidity. As a result, battery performance may be greatly improved.

Based on 100 % by weight in total of the electrolyte solution, the electrolyte solution of the present invention may include the first additive in an amount of 20 % by weight or less, preferably 0.01 to 15 % by weight. Within this range, decomposition of the electrolyte solution may be suppressed, thereby improving the lifespan characteristics and cycle characteristics of the battery.

For example, the second additive may have an atomic group represented by Formula 2 below. In this case, the second additive may be adsorbed on the metal surface of an electrode and may inhibit side reactions between the electrode and an electrolyte, thereby further improving the stability of the electrolyte solution and thus improving the cycle characteristics, stability, and lifespan of the battery.

[Formula 2]

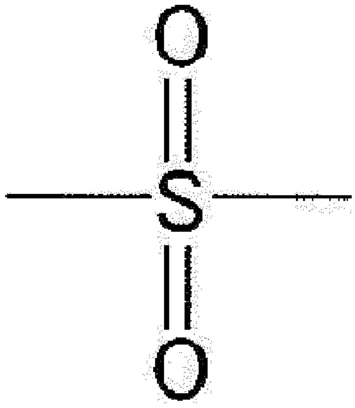

In Formula 2, a solid line is a bond.

As used herein, the term "atomic group" refers to a covalently bonded polyatomic ion, unless otherwise specified.

The second additive may be a compound represented by Formula 5 below.

[Formula 5]

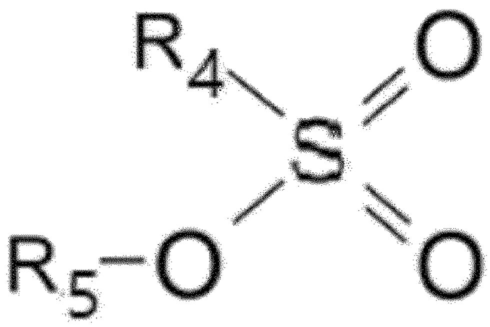

In Formula 5, $R_4$ is $OR_6$ or $R_6$; $R_5$ is $R_7A$; A is

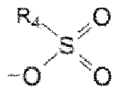;

and $R_6$ and $R_7$ are independently an alkyl group having 1 to 5 carbon atoms or an aryl group having 6 to 10 carbon atoms.

When an electrolyte solution additive including the compound represented by Formula 5 is added to the electrolyte solution of a secondary battery, electrons are localized toward O element due to the electronegativity difference between S element and O element directly bonded thereto. Accordingly, S element becomes electron-poor ($e^-$ poor, $\delta+$) state, and oxidation reaction is induced in the electrolyte solution containing lithium ions, thereby forming a stable film on an electrode, specifically, a cathode. In addition, ionic conductivity is improved due to a halogen substituent substituted at the terminal.

At this time, decomposition of the electrolyte solution may be prevented due to the stability of the film, and thus cycle characteristics may be improved. In particular, compared to conventional electrode films that decompose at high temperatures and have poor high-temperature storage properties, the film does not decompose at high temperatures and thus has excellent high-temperature storage properties. In addition, resistance increase may be prevented, and thus charging efficiency and output may be improved. In addition, the safety of a battery may be improved because gas generation due to chemical reaction inside the battery is suppressed.

Specifically, gas generation inside a battery is mainly due to decomposition of electrolyte solution components, especially carbonate-based solvents, on the surface of a cathode/anode. A cathode/anode protective film may be easily degraded by the generated gas, and gas generation is further promoted due to oxygen radicals generated from the

cathode. The material of the present invention may suppress direct decomposition of a solvent by forming a protective film composed of S, O, and F components and having excellent stability. In addition, the material of the present invention may prevent elution of transition metal ions from the cathode due to cathode deterioration due to the metal ion coordination effect, thereby ultimately preventing escape of oxygen elements constituting the skeleton of the cathode.

In addition, structural collapse of the electrode active materials of a cathode and anode may be prevented at high temperatures, thereby improving capacity retention. As a result, the effect of increasing a lifespan may be obtained.

The second additive may be a compound represented by Formula 6 below. When a lithium secondary battery is manufactured using the electrolyte solution including the second additive, decomposition of the electrolyte solution may be suppressed by the terminal group of the compound, thereby reducing an internal resistance increase rate, gas generation, and a thickness increase rate. As a result, a battery lifespan may be increased.

[Formula 6]

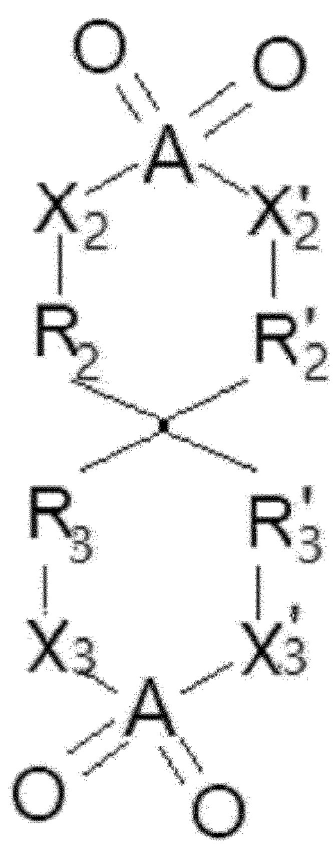

In Formula 6, A is phosphorus (P), sulfur (S), or nitrogen (N); $X_1$, $X_2$, $X_3$, $X_1$', $X_2$', and $X_3$' are independently a bond or oxygen; n is an integer from 0 to 3; and $R_2$, $R_2$, $R_3$, $R_1$', $R_2$', and $R_3$' are independently a bond or a substituted or unsubstituted alkylene having 1 to 10 carbon atoms and include one or more carbon atoms.

As a specific example, when the second additive has the structure represented by Formula 6, due to the symmetrical structure, flow of electrons in a molecule may be stabilized. As a result, battery performance may be greatly improved.

The compound represented by Formula 6 may be preferably a compound represented by Formula 7 below. In this case, due to the symmetrical structure, flow of electrons in a molecule may be stabilized. As a result, battery performance may be greatly improved.

[Formula 7]

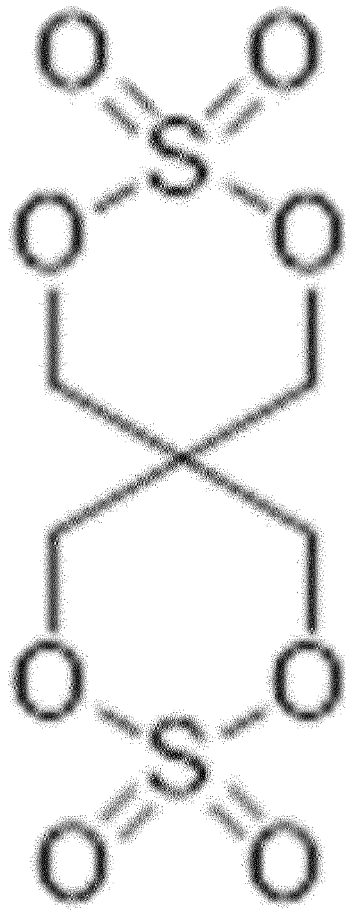

In Formula 7, a solid line is a bond; carbon is located at a point where no separate element is shown and bonds meet; and the number of hydrogens satisfying a valence of the carbon is omitted.

Based on 100 % by weight in total of the electrolyte solution, the electrolyte solution of the present invention may include the second additive in an amount of 0.1 to 10 % by weight, preferably 0.1 to 5 % by weight. Within this range, decomposition of the electrolyte solution may be suppressed, thereby improving the lifespan characteristics and cycle characteristics of the battery.

When the electrolyte solution of the present invention is prepared using the first and second additives in combination, the effect of suppressing side reactions of an electrolyte may be maximized. When a lithium secondary battery is manufactured using the electrolyte solution, gas generation may be reduced when left at high temperatures, thereby decreasing internal resistance increase rate and ultimately improving the lifespan characteristics of the battery.

Based on 100 % by weight in total of the electrolyte solution, the first and second additives may be included in a weight ratio (first additive:second additive) of 1:0.1 to 1.7 or 1:0.2 to 1.6. In this case, battery stability may be improved.

A non-aqueous solvent included in the electrolyte solution of the present invention is not particularly limited as long as the solvent is capable of minimizing decomposition due to oxidation reaction during the charging and discharging process of the battery and obtaining the desired characteristics in combination with the additives. For example, the non-aqueous solvent may be a carbonate-based organic solvent, an acetate-based organic solvent, a propionate-based organic solvent, or an ester-based organic solvent. The solvent may be used alone, or the two or more solvents may be used in combination.

For example, 10 to 100 % by volume of the carbonate-based organic solvent and up to 90 % by volume of one or more selected from the group consisting of the acetate-based organic solvent, the propionate-based organic solvent, and the ester-based organic solvent may be mixed and used.

For example, among the non-aqueous solvents, the carbonate-based organic solvent may include one or more selected from the group consisting of ethylene carbonate (EC), propylene carbonate (PC), butylene carbonate (BC), vinylene carbonate (VC), dimethyl carbonate (DMC), diethyl carbonate (DEC), dipropyl carbonate (DPC), ethyl methyl carbonate (EMC), methyl ethyl carbonate (MEC), fluoroethylene carbonate (FEC), methyl propyl carbonate (MPC), and ethyl propyl carbonate (EPC) .

More preferably, among the carbonate-based organic solvents, a high-permittivity carbonate-based organic solvent capable of improving battery charge/discharge performance and having high ionic conductivity and a low-viscosity carbonate-based organic solvent capable of properly adjusting the viscosity of the high-permittivity organic solvent may be mixed and used.

Specifically, a high-permittivity organic solvent selected from the group consisting of ethylene carbonate, propylene carbonate, and a mixture thereof and a low-viscosity organic solvent selected from the group consisting of ethyl methyl carbonate, dimethyl carbonate, diethyl carbonate, and a mixture thereof may be mixed and used.

Preferably, the high-permittivity organic solvent and the low-viscosity organic solvent may be mixed in a volume ratio of 2:8 to 8:2. More specifically, ethylene carbonate or propylene carbonate; ethyl methyl carbonate; and dimethyl carbonate or diethyl carbonate may be mixed in a volume ratio of 5:1:1 to 2:5:3, as a specific example, a volume ratio of 3:5:2.

As a specific example, the carbonate-based organic solvent may include ethylene carbonate (EC), diethyl carbonate (DEC), and ethyl methyl carbonate (EMC). In this case, 10 to 40 % by weight, 15 to 35 % by weight, 20 to 30 % by weight, or 22 to 28 % by weight of ethylene carbonate; 15 to 45 % by weight, 20 to 40 % by weight, 25 to 35 % by weight, or 27 to 33 % by weight of diethyl carbonate; and 30 to 60 % by weight, 35 to 55 % by weight, 40 to 50 % by weight, or 42 to 48 % by weight of ethyl methyl carbonate may be mixed and used.

For example, the acetate-based organic solvent may include methyl acetate, ethyl acetate, and propyl acetate, without being limited thereto.

For example, the propionate-based organic solvent may include propionate and methyl propionate, without being limited thereto.

The ester-based organic solvent may be $\gamma$-butyrolactone, $\gamma$-valerolactone, $\gamma$- caprolactone, $\sigma$-valerolactone, $\varepsilon$-caprolactone, or the like.

For example, the organic solvent may be used in the remaining amount after subtracting the content of components other than the organic solvent in the electrolyte solution. As a specific example, based on a total weight of the electrolyte solution, the organic solvent may be used in an amount of 10 % by weight or less.

When the organic solvent contains moisture, lithium ions in the electrolyte solution may be hydrolyzed. Accordingly, the amount of moisture in the organic solvent is preferably adjusted to 150 ppm or less, more preferably 100 ppm or less.

For example, lithium salts that may be included in the electrolyte solution of the present invention may include one or more selected from the group consisting of $LiPF_6$, $LiClO_4$, LiTFSI, $LiAsF_6$, $LiBF_4$, $LiBF_6$, $LiSbF_6$, $LiAl0_4$, $LiAlCl_4$, LiBOB, $LiB_{10}Cl_{10}$, $LiAlCl_4$, $LiAlO_4$, $LiClO_4$, $LiCF_3SO_3$, $LiCH_3CO_2$, $LiCF_3CO_2$, $LiAsF_6$, $LiSbF_6$, $LiCH_3SO_3$, LiBeTI, $LiC_4F_9SO_3$, $LiN(C_2F_5SO_3)_2$, $LiN(C_2F_5SO_2)_2$, $LiN(CF_3SO_2)_2$, $LiN(CaF_{2a+1}SO_2)(C_bF_{2b+1}SO_2)$ (a and b are natural numbers), LiCl, LiI, LiBr, and $LiB(C_2O_4)_2$, preferably $LiPF_6$. For example, a and b may be integers from 1 to 4.

When the lithium salt is dissolved in an electrolyte solution, the lithium salt functions as a source of lithium ions in a lithium secondary battery and may promote movement of lithium ions between a cathode and an anode. Accordingly, the lithium salt is preferably included in the electrolyte solution at a concentration of about 0.6 to 2 M. When the concentration of the lithium salt is less than 0.6 M, the conductivity of the electrolyte may be reduced, resulting in poor electrolyte performance. When the concentration of the lithium salt exceeds 2 M, the viscosity of the electrolyte may increase, which reduces the mobility of lithium ions.

Considering the conductivity of the electrolyte and the mobility of lithium ions, the lithium salt may be included in the electrolyte solution at a concentration of 0.5 to 1.5 M (mol/L), preferably 0.7 to 1.3 M, more preferably 0.8 to 1.1 M. Within this range, the performance of the electrolyte solution may be excellent due to high conductivity of the electrolyte solution, and the mobility of lithium ions may be excellent due to low viscosity of the electrolyte solution.

For example, the electrolyte solution may have a lithium ion conductivity of 0.3 S/m or more or 0.3 to 10 S/m at 25 °C. Within this range, the cycle characteristics and lifespan characteristics of the lithium secondary battery may be further improved.

In addition to the components thereof, the electrolyte solution may further include electrolyte solution additives that are generally used in electrolyte solutions for the purpose of improving the lifespan characteristics of a battery, suppressing a decrease in battery capacity, and improving the discharge capacity of a battery.

For example, the electrolyte solution additives may include one or more (hereinafter also referred to as "third additive") selected from the group consisting of boron compounds, phosphorus compounds, sulfur compounds, and nitrogenous compounds.

For example, the third additive may include one or more selected from compounds represented by Formulas 8 to 19 below. In this case, decomposition of the electrolyte may be suppressed more effectively.

[Formula 8]

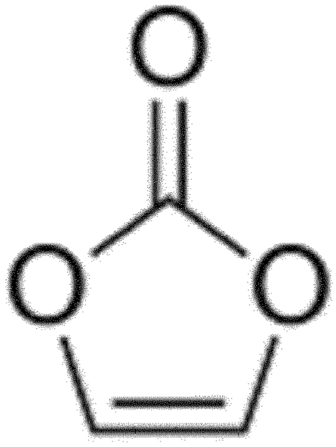

[Formula 9]

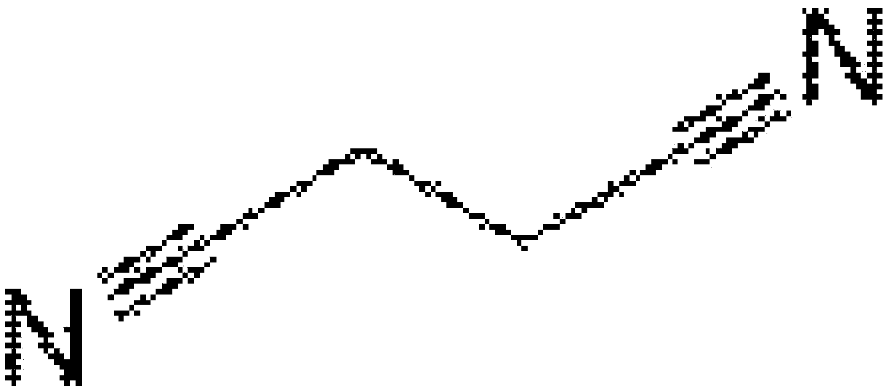

[Formula 10]

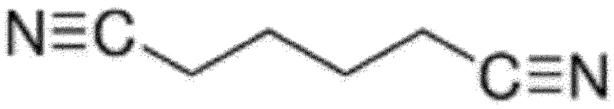

[Formula 11]

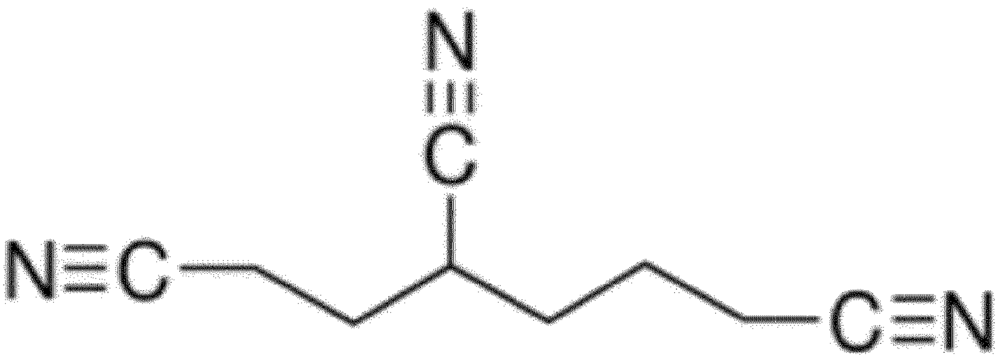

[Formula 12]

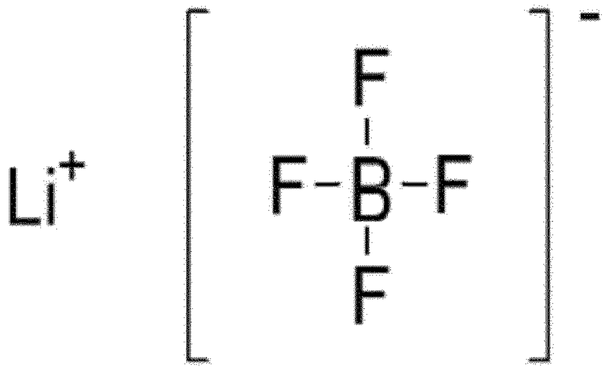

[Formula 13]

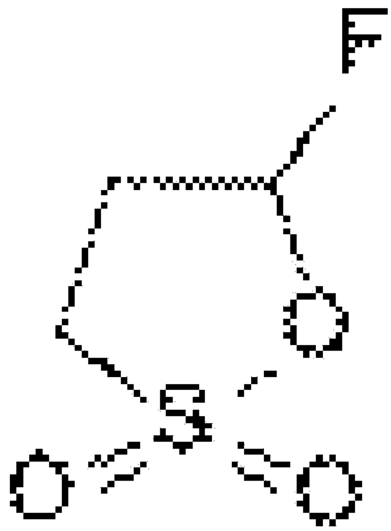

[Formula 14]

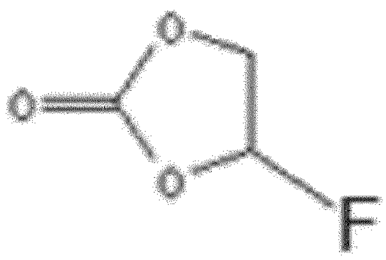

[Formula 15]

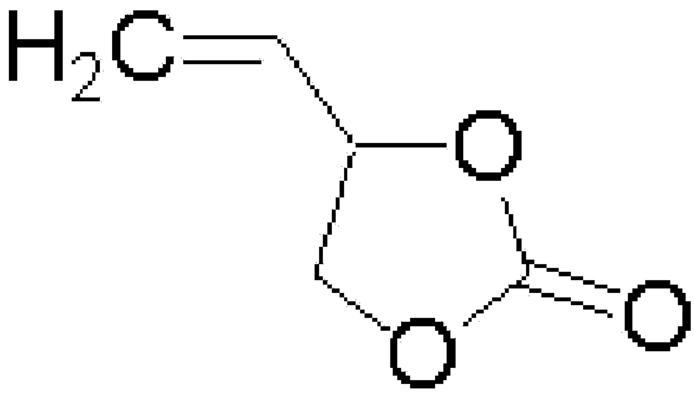

[Formula 16]

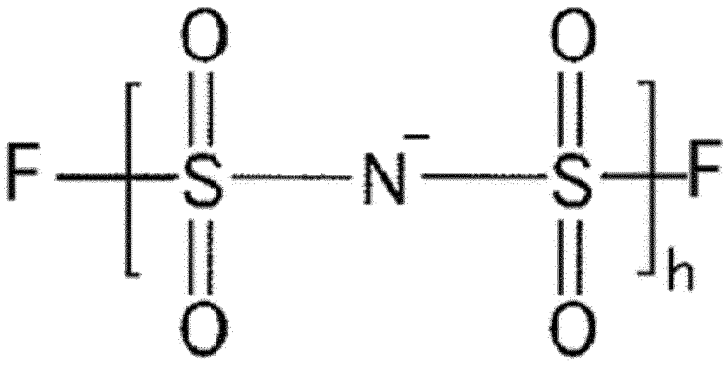

[Formula 17]

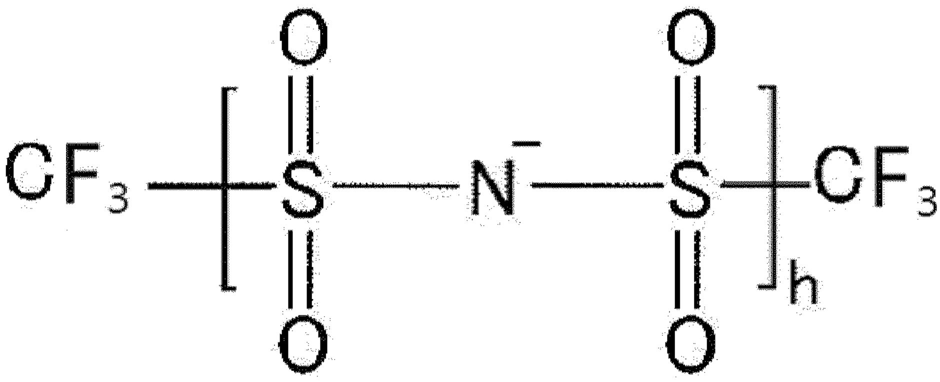

[Formula 18]

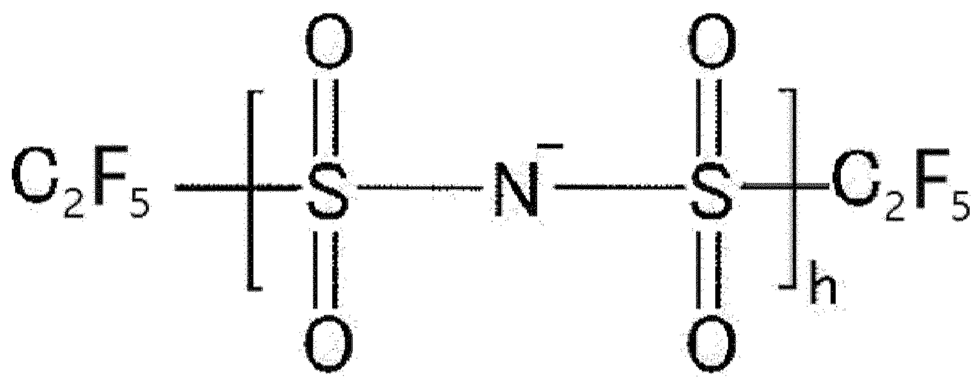

[Formula 19]

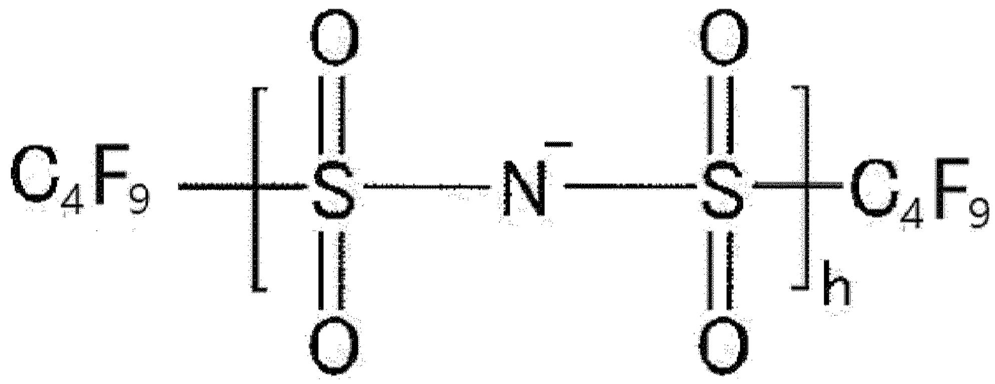

In Formulas 8 to 19, a solid line is a bond; carbon is located at a point where no separate element is shown and bonds meet; and the number of hydrogens satisfying a valence of the carbon is omitted.

Based on 100 % by weight in total of the electrolyte solution, the electrolyte solution of the present invention may include the third additive in an amount of 10 % by weight or less, preferably 1 to 5 % by weight. Within this range, decomposition of the electrolyte solution may be suppressed, thereby improving the lifespan characteristics and cycle characteristics of the battery.

In addition, the electrolyte solution additives may include, for example, a metal fluoride. When the electrolyte solution additives further include the metal fluoride, the influence of acids generated around a cathode active material may be reduced, the reaction between the cathode active material and the electrolyte solution may be suppressed, and rapid decrease in the capacity of the battery may be suppressed.

Specifically, the metal fluoride may include one or more selected from the group consisting of LiF, RbF, TiF, AgF, $AgF_2$, $BaF_2$, $CaF_2$, $CdF_2$, $FeF_2$, $HgF_2$, $Hg_2F_2$, $MnF_2$, $NiF_2$, $PbF_2$, $SnF_2$, $SrF_2$, $XeF_2$, $ZnF_2$, $AlF_3$, $BF_3$, $BiF_3$, $CeF_3$, $CrF_3$, $DyF_3$, $EuF_3$, $GaF_3$, $GdF_3$, $FeF_3$, $HoF_3$, $InF_3$, $LaF_3$, $LuF_3$, $MnF_3$, $NdF_3$, $PrF_3$, $SbF_3$, $ScF_3$, $SmF_3$, $TbF_3$, $TiF_3$, $TmF_3$, $YF_3$, $YbF_3$, $TlF_3$, $CeF_4$, $GeF_4$, $HfF_4$, $SiF_4$, $SnF_4$, $TiF_4$, $VF_4$, $ZrF_4$, $NbF_5$, $SbF_5$, $TaF_5$, $BiF_5$, $MoF_6$, $ReF_6$, $SF_6$, $WF_6$, $CoF_2$, $CoF_3$, $CrF_2$, CsF, $ErF_3$, $PF_3$, $PbF_3$, $PbF_4$, $ThF_4$, $TaF_5$, and $SeF_6$.

For example, based on a total weight of the electrolyte solution, the metal fluoride may be included in an amount of 0.1 to 10 % by weight or 0.2 to 5 % by weight. Within this range, the cycle characteristics and lifespan characteristics of the lithium secondary battery may be further improved.

In the electrolyte solution according to the present invention having the above composition ratio, decomposition reaction of the electrolyte solution is inhibited in a wide temperature range from -20 °C to 60 °C, reducing gas generation and internal resistance increase rate. Thus, a secondary battery including the electrolyte solution having high stability and reliability may be provided. In addition, since the structure of the battery is the same as that of a general secondary battery, the battery may be easily manufactured and is advantageous for mass production.

The lithium secondary battery according to the present invention may include a cathode; an anode; a separator disposed between the cathode and the anode; and an electrolyte solution. The electrolyte solution may include the above-described electrolyte solution, and the cathode and the anode may include a cathode active material and an

anode active material, respectively.

For example, the cathode may be prepared by obtaining a composition for forming a cathode active material layer by mixing a cathode active material, a binder, and optionally a conductive agent, and then applying the composition to a cathode current collector such as aluminum foil.

For example, as the cathode active material, common lithium composite metal oxides and lithium olivine-type phosphates used in lithium secondary batteries may be used. Preferably, the cathode active material may include one or more selected from the group consisting of cobalt, manganese, nickel, and iron, more preferably a lithium nickel manganese cobalt oxide (NCM).

As a specific example, the cathode active material may be a lithium composite metal oxide represented by a chemical formula of $Li[Ni_xCo_{1-x-y}Mn_y]O_2$ ($0<x<0.5$, $0<y<0.5$), but the present invention is not limited thereto. For example, in the Formula of $Li[Ni_xCo_{1-x-y}Mn_y]O_2$ representing the lithium composite metal oxide, the variables x and y may be $0.0001<x<0.5$, $0.0001<y<0.5$, or $0.001<x<0.3$, $0.001<y<0.3$.

As another example, a compound (lithiated intercalation compound) capable of reversible intercalation and deintercalation of lithium may be used as the cathode active material. To improve the capacity characteristics and stability of a battery, among the compounds, one or more selected from the group consisting of $LiCoO_2$, $LiMnO_2$, $LiMn_2O_4$, $LiNiO_2$, $LiNi_xMn_{(1-x)}O_2$ ($0<x<1$), and $LiMl_xM2_yO_2$ ($0\leq x\leq 1$, $0\leq y\leq 1$, $0\leq x+y\leq 1$, and M1 and M2 are each independently any one selected from the group consisting of Al, Sr, Mg, and La) are preferably used.

In addition, for example, among the cathode active materials, the lithium olivine-type phosphate may include one or more selected from iron, cobalt, nickel, and manganese, specifically, $LiFePO_4$, $LiCoPO_4$, and $LiMnPO_4$. In addition, compounds in which some metals in the lithium olivine-type phosphate are replaced with other metals may also be used.

For example, the anode may be prepared by obtaining a composition for forming an anode active material layer by mixing an anode active material, a binder, and optionally a conductive agent, and then applying the composition to an anode current collector such as copper foil.

For example, as the anode active material, a compound capable of reversible intercalation and deintercalation of lithium may be used.

For example, the anode active material may include one or more selected from the group consisting of tin, tin compounds, silicon, silicon compounds, lithium titanate, crystalline carbon, amorphous carbon, synthetic graphite, and natural graphite.

In the present disclosure, the tin compound is a compound obtained by combining tin with one or more other chemical elements, and the silicon compound is a compound obtained by combining silicon with one or more other chemical elements.

In addition to the carbonaceous materials such as crystalline carbon, amorphous carbon, and graphite, a metallic compound capable of alloying with lithium or a composite including a metallic compound and a carbonaceous material may also be used as the anode active material. For example, graphite may be used as the anode active material.

For example, as the metal capable of alloying with lithium, any one of Si, Al, Sn, Pb, Zn, Bi, In, Mg, Ga, Cd, Si alloy, Sn alloy, and Al alloy may be used. In addition, a metallic lithium thin film may be used as the anode active material.

As the anode active material, at least one selected from the group consisting of crystalline carbon, amorphous carbon, carbon composites, lithium metal, and lithium-containing alloys, known to have high stability, may be used.

For example, a battery assembly may be manufactured by placing a separator between the aforementioned cathode and anode, inserting the separator/cathode/anode assembly into a cell, injecting the electrolyte solution thereto, and sealing the cell. For example, the lithium secondary battery including the above-described electrolyte solution, cathode, anode, and separator may be formed in a unit cell having a structure of cathode/separator/anode, a bi-cell having a structure of cathode/separator/anode/separator/cathode, or a structure of a laminated cell in which the structure of a unit cell is repeated.

In the secondary battery according to one embodiment of the present invention, by adding the first and second additives to improve battery performance, battery characteristics such as battery discharge resistance, output characteristics, capacity recovery characteristics at high temperatures of 60 °C or higher, and lifespan characteristics measured by HPPC (Hybrid Pulse Power Characterization) may be improved.

In the secondary battery according to another embodiment of the present invention, by adding a performance improver in addition to the first additive and second additives added to the electrolyte solution, battery characteristics such as battery discharge resistance, output characteristics, capacity recovery characteristics at high temperatures of 60 °C or higher, and lifespan characteristics measured by HPPC (Hybrid Pulse Power Characterization) may be further improved.

Specifically, the secondary battery of the present invention may have an HPPC discharge resistance value of 40 mΩ or less, as a specific example, 25 to 39 mΩ as measured at 60 °C.

In the present disclosure, the HPPC discharge resistance value may be measured by the method prescribed in the literature "Battery test manual for plug-in hybrid electric vehicles (2010, Idaho National Laboratory for the U.S. Department of Energy)", and is an important index representing the output characteristics of a battery. In addition, the

discharge resistance is a resistance value measured during discharging of a battery. Within the range, improved output performance may be provided. As the discharge resistance decreases, energy loss is reduced, thereby increasing a charging speed and thus improving the output of a battery. The HPPC discharge resistance value of the secondary battery of the present invention is reduced up to 23.6 %. Due to these advantages, the secondary battery of the present invention has high charging speed and output, and thus is suitable for use as an automobile battery.

The secondary battery of the present invention may have a recovery capacity of 1,000 mAh or more, as a specific example, 1,000 to 1,130 mAh as measured at 60 °C.

In the present disclosure, the recovery capacity represents the capacity retention characteristics of a battery that has been left unattended for a long period of time. The recovery capacity may be obtained by measuring and comparing discharged electric capacity when a battery left for a long time is discharged to a discharge final voltage and discharged electric capacity when the discharged battery is recharged and discharged again to the discharge final voltage. As the recovery capacity increases, the amount of spontaneous discharge due to battery preservation (storage) decreases, which means that a battery may be preserved for a long time. In particular, as the storage temperature of a battery increases, spontaneous discharge rate increases. Thus, recovery capacity at high temperatures is a very important characteristic of automotive batteries. When the electrolyte solution additives of the present invention are added to an electrolyte solution for batteries, the recovery capacity is increased as described above, and thus long-term storage is possible with only one charge.

In addition, the secondary battery may have a thickness of 4 mm or less, preferably 3.9 to 4.1 mm as measured at 60 °C.

In addition, the secondary battery may have a thickness increase rate of 10.81 % or less, preferably 2.63 to 10.81 % as measured at 60 °C and calculated by Equation 1 below.

[Equation 1]

$$\text{Thickness increase rate (\%)} = \{(\text{Thickness after storage at high temperature} - \text{Initial thickness}) / \text{Initial thickness}\} \times 100$$

In the present disclosure, the thickness increase rate represents the swelling characteristics due to gas generation inside a battery, and may be obtained by measuring the initial thickness of a pouch cell and the thickness after leaving at high temperature and comparing the difference between the two values. When a stable film is formed on the cathode and anode, decomposition of electrolyte components may be suppressed. Accordingly, when the thickness increase rate is reduced, stability according to repeated charging and discharging of a battery may be provided, and battery lifespan may be improved.

In addition, the secondary battery may have a coulomb efficiency of 99.4 % or more, preferably 99.4 to 99.8 % as calculated by Equation 2 below.

[Equation 2]

$$\text{Coulomb efficiency (\%)} = (\text{Discharge capacity at 300 cycles} / \text{charge capacity at 300 cycles}) \times 100$$

In the present disclosure, the coulomb efficiency is calculated based on charge capacity and discharge capacity at 300 cycles. The coulomb efficiency is associated with an effect of improving charging and discharging efficiency.

Therefore, when the battery of the present invention is used as a battery for automobiles, output dependent on the size of an automobile may be improved. In addition, performance at low and high temperatures associated with climate change and direct exposure to sunlight while driving or parked may be improved, and thus lifespan may be increased. Thus, the battery of the present invention may exhibit excellent performance as an automobile battery.

The lithium secondary battery according to the present invention may be classified into a lithium ion battery, a lithium ion polymer battery, and a lithium polymer battery according to the types of separator and electrolyte solution

used. In addition, the lithium secondary battery of the present invention may be classified into cylindrical, prismatic, coin, and pouch types according to the shape thereof. In addition, the lithium secondary battery of the present invention may be classified into a bulk type and a thin-film type according to the size thereof. Thereamong, the electrolyte solution according to an embodiment of the present invention may be suitably applied to a lithium ion battery, an aluminum laminated battery, and a lithium polymer battery.

The lifespan characteristics of the lithium secondary battery including the electrolyte solution according to the present invention may be improved at a high temperature of 45 °C or higher, as a specific example, 45 °C to 60 °C, and the internal resistivity thereof may be increased. In addition, in the lithium secondary battery, thickness increase rate and gas generation may be reduced. Thus, when the battery of the present invention is used as an automobile battery, output associated with the size of an automobile and performance at low and high temperatures associated with climate change and direct exposure to sunlight while driving or parked may be improved. Thus, the battery of the present invention may exhibit excellent performance as an automobile battery.

That is, when the electrolyte solution additives according to embodiments of the present invention and the electrolyte solution including the same are used to manufacture a secondary battery, the discharge resistance, output, recovery capacity, and lifespan efficiency of the secondary battery may be improved, and thus the secondary battery is suitable for use as a secondary battery for automobiles. In addition, the secondary battery may be useful for portable devices such as mobile phones, notebook computers, digital cameras, and camcorders, electric vehicles such as hybrid electric vehicles (HEVs) and plug-in HEVs (PHEVs), and medium- and large-sized energy storage systems.

Hereinafter, the present invention will be described in more detail with reference to the following preferred examples. However, these examples are provided for illustrative purposes only and should not be construed as limiting the scope and spirit of the present invention. In addition, it will be apparent to those skilled in the art that various changes and modifications may be made without departing from the spirit and scope of the present invention, and such changes and modifications are also within the scope of the appended claims.

**[Examples: Preparation of electrolyte solution for batteries]**

**Examples 1-1 and 1-2, Comparative Example 1-1, and Reference Example 1-1**

An organic solvent was prepared by dissolving, at a concentration of 1.15 M, $LiPF_6$ as a lithium salt in a carbonate-based mixed solvent containing EC, EMC, and DEC in a volume ratio of 2:4:4 (EC:EMC:DEC), and an electrolyte solution for batteries was prepared by mixing a compound represented by Formula 1a below (or Formula 8) as the first electrolyte solution additive, a compound represented by Formula 1b below (or Formula 15), a compound represented by Formula 1c below, and a compound represented by Formula 2a below (or Formula 7) as the second electrolyte solution additive according to the contents shown in Table 1 below.

[Formula 1a]

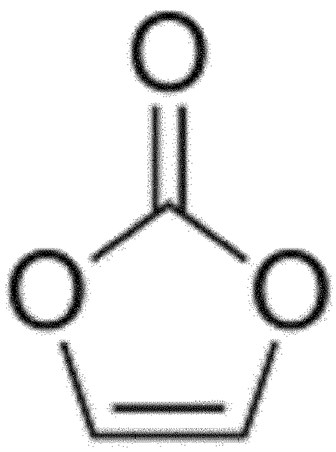

[Formula 1b]

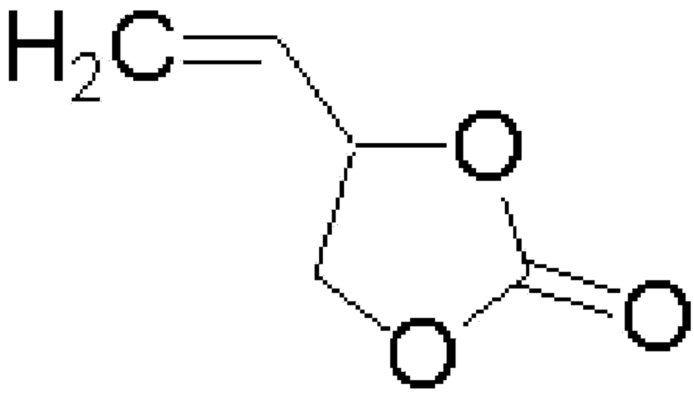

[Formula 1c]

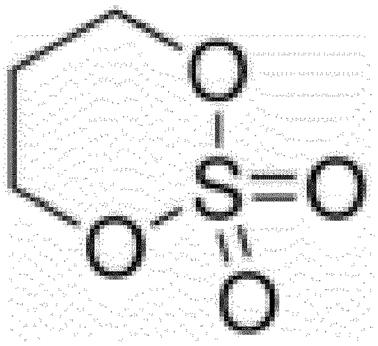

[Formula 2a]

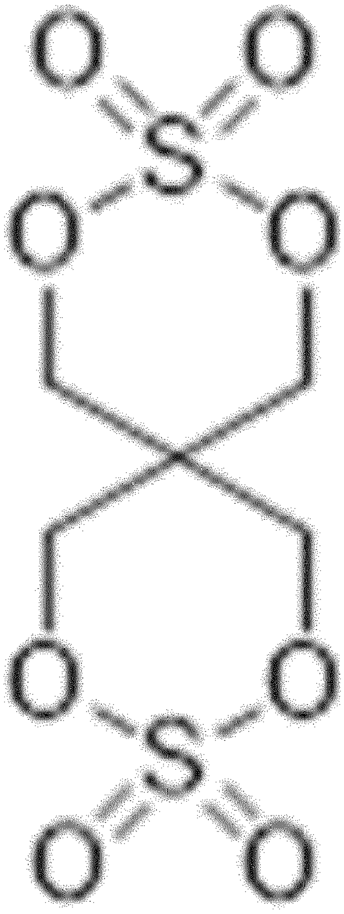

[Table 1]

| Classification | Type of first additive | Content of first additive (wt%) | Type of second additive | Content of second additive (wt%) |
|---|---|---|---|---|
| Example 1-1 | Formula 1a | 5 | Formula 2a | 3 |
| Example 1-2 | Formula 1a | 5 | Formula 2a | 3 |
| | Formula 1b | 5 | | |
| Comparative Example 1-1 | Formula 1a | 5 | - | - |
| Reference Example 1-1 | Formula 1a | 5 | Formula 2a | 3 |
| | Formula 1c | 10 | | |

## Examples 2-1 to 2-3 and Comparative Example 2-1

An organic solvent was prepared by dissolving, at a concentration of 1.15 M, $LiPF_6$ as a lithium salt in a carbonate-based mixed solvent containing EC, EMC, and DMC in a volume ratio of 2:4:4 (EC:EMC:DMC), and an electrolyte solution for batteries was prepared by mixing a compound represented by Formula 1d below (or Formula 13) as the first electrolyte solution additive, the compound represented by Formula 1a (or Formula 8), the compound represented by Formula 1b (or Formula 15), and the compound represented by Formula 2a as the second electrolyte solution additive according to the contents shown in Table 2 below.

[Formula 1d]

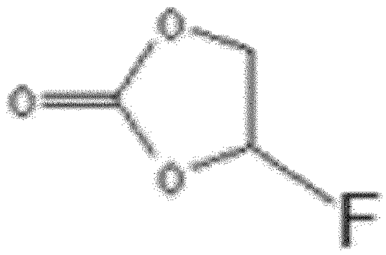

[Table 2]

| Classification | Type of first additive | Content of first additive (wt%) | Type of second additive | Content of second additive (wt%) |
|---|---|---|---|---|
| Example 2-1 | Formula 1d | 10 | Formula 2a | 3 |
| Example 2-2 | Formula 1d | 5 | Formula 2a | 3 |
| | Formula 1b | 5 | | |
| Example 2-3 | Formula 1d | 5 | Formula 2a | 3 |
| | Formula 1a | 5 | | |
| | Formula 1b | 5 | | |
| Comparative Example 2-1 | Formula 1d | 5 | - | - |

**Example 3-1, Comparative Example 3-1, and Reference Example 3-1**

An organic solvent was prepared by dissolving, at a concentration of 1.15 M, $LiPF_6$ as a lithium salt in a carbonate-based mixed solvent containing EC, EMC, and DEC in a volume ratio of 2:4:4 (EC:EMC:DEC), and an electrolyte solution for batteries was prepared by mixing the compound represented by Formula 1b (or Formula 18) as the first electrolyte solution additive and the compound represented by Formula 2a as the second electrolyte solution additive according to the contents shown in Table 3 below.

[Table 3]

| Classification | Type of first additive | Content of first additive (wt%) | Type of second additive | Content of second additive (wt%) |
|---|---|---|---|---|
| Example 3-1 | Formula 1b | 5 | Formula 2a | 3 |
| Comparative Example 3-1 | Formula 1b | 5 | - | - |
| Reference Example 3-1 | Formula 1b | 3 | Formula 2a | 3 |

**Examples 4-1 and 4-2 and Comparative Examples 4-1 and 4-2**

An organic solvent was prepared by dissolving, at a concentration of 1.15 M, $LiPF_6$ as a lithium salt in a carbonate-based mixed solvent containing EC, EMC, and DMC in a volume ratio of 2:4:4 (EC:EMC:DMC), and an electrolyte solution for batteries was prepared by mixing a compound represented by Formula 1e below (or Formula 3) as the first electrolyte solution additive, a compound represented by Formula 1f below (or Formula 4), a compound represented by Formula 1g below, and the compound represented by Formula 2a as the second electrolyte solution

additive according to the contents shown in Table 4 below.

[Formula 1e]

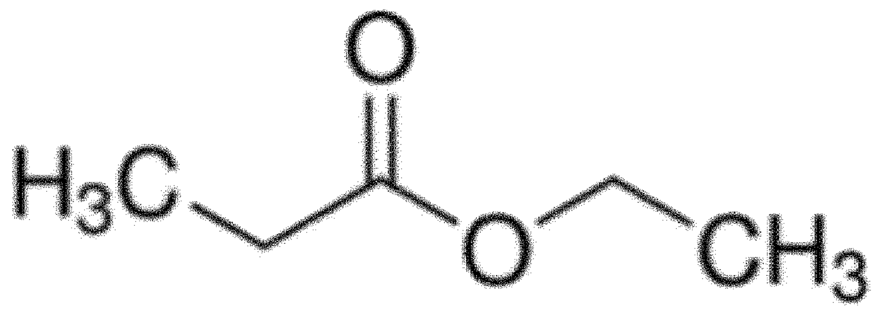

[Formula 1f]

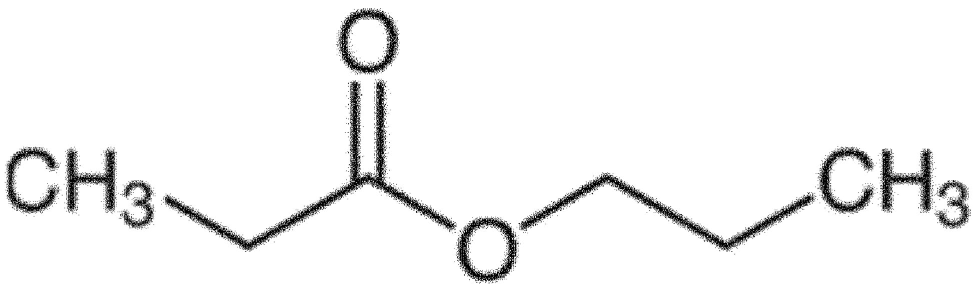

[Formula 1g]

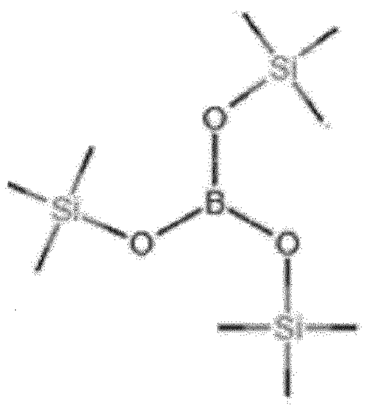

[Table 4]

| Classification | Type of first additive | Content of first additive (wt%) | Type of second additive | Content of second additive (wt%) |
|---|---|---|---|---|
| Example 4-1 | Formula 1e | 15 | Formula 2a | 3 |
| Example 4-2 | Formula 1e | 10 | Formula 2a | 3 |
| | Formula 1f | 10 | | |
| Comparative Example 4-1 | Formula 1e | 10 | Formula 2a | 3 |
| | Formula 1g | 5 | | |

(continued)

| Classification | Type of first additive | Content of first additive (wt%) | Type of second additive | Content of second additive (wt%) |
|---|---|---|---|---|
| Comparative Example 4-2 | Formula 1e | 15 | - | - |

**Example 5-1 and Comparative Examples 5-1 to 5-3**

An organic solvent was prepared by dissolving, at a concentration of 1.15 M, $LiPF_6$ as a lithium salt in a carbonate-based mixed solvent containing EC, EMC, and DEC in a volume ratio of 2:4:4 (EC:EMC:DEC), and an electrolyte solution for batteries was prepared by mixing a compound represented by Formula 1h below (or Formula 16) as the first electrolyte solution additive, wherein h is 1 and a cation is lithium, a compound represented by Formula 1h below, wherein h is 1 and a cation is sodium, the compound represented by Formula 1g, and the compound represented by Formula 2a as the second electrolyte solution additive according to the contents shown in Table 5 below.

[Formula 1h]

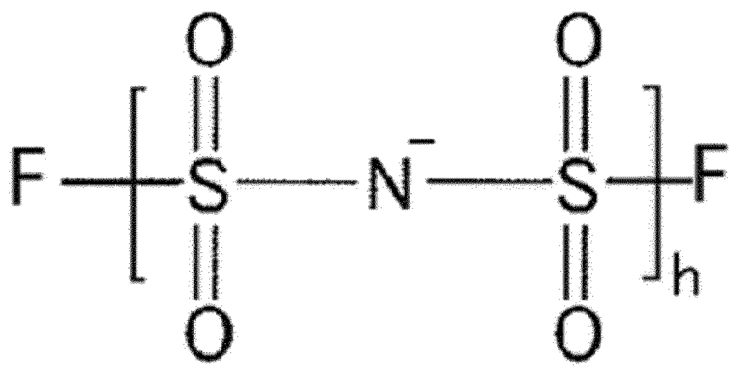

[Table 5]

| Classification | Type of first additive | Content of first additive (wt%) | Type of second additive | Content of second additive (wt%) |
|---|---|---|---|---|
| Example 5-1 | Formula 1h + lithium | 0.5 | Formula 2a | 3 |
| Comparative Example 5-1 | Formula 1h + lithium | 10 | - | - |
| Comparative Example 5-2 | Formula 1h + sodium | 0.5 | Formula 2a | 3 |
| Comparative Example 5-3 | Formula 1h + lithium | 10 | Formula 2a | 3 |
| | Formula 1g | 5 | | |

**Manufacture of battery**

100 parts by weight of a cathode mixture containing 92 % by weight of Li $(Ni_{0.8}Co_{0.1}Mn_{0.1})O_2$ as a cathode active material, 4 % by weight of carbon black as a conductive agent, and 4 % by weight of polyvinylidene fluoride (PVdF) as a binder was added to 100 parts by weight of N-methyl-2-pyrrolidone (NMP) as a solvent to prepare cathode mixture slurry. The cathode mixture slurry was applied to an aluminum (Al) thin film having a thickness of about 20 um as a cathode current collector. Then, the thin film was dried, and then was subjected to roll press to manufacture a cathode.

In addition, 100 parts by weight of an anode mixture containing 96 % by weight of carbon powder blended with

artificial graphite and natural graphite as an anode active material, 3 % by weight of PVdF as a binder, and 1 % by weight of carbon black as a conductive agent was added to 100 parts by weight of NMP as a solvent to prepare anode mixture slurry. The anode mixture slurry was applied to a copper (Cu) thin film having a thickness of 10 μm as an anode current collector. Then, the thin film was dried, and then subjected to roll press to manufacture an anode.

A pouch-type battery was manufactured according to a conventional method using the manufactured cathode and anode and a separator composed of three layers of polypropylene/polyethylene/polypropylene (PP/PE/PP). Then, the electrolyte solutions prepared in Examples 1-1 to 5-1, Comparative Examples 1-1 to 5-3, and Reference Examples 1-1 and 3-1 were injected into the pouch-type battery to manufacture lithium secondary batteries.

## Test Examples

The performance of the manufactured secondary batteries was evaluated according to the following methods, and the results are shown in Tables 6 and 7 below.

[Evaluation of HPPC discharge resistance]

HPPC discharge resistance was measured according to the method prescribed in the literature "Battery test manual for plug-in hybrid electric vehicles (2010, Idaho National Laboratory for the U.S. Department of Energy)".

After standing for 5 hours at 60 °C, a measurement voltage value, a charge/discharge current value corresponding to C-rate, current change (ΔI), discharge voltage change (ΔV), charge voltage change (ΔV), discharge resistance, and charge resistance were measured. A resistance value was calculated using a slope value obtained from change in current and voltage by briefly flowing charge/discharge current for each C-rate for a certain period of time, and the results are shown in the initial discharge resistance item in Tables 6 and 7 below.

[Evaluation of high-temperature recovery capacity]

According to the charging conditions, charging was performed at a constant current of 1.0 C and a voltage of 4.2 V until charge current became 1/10 C. According to the discharging conditions, after performing charging and discharging by discharging to 3.0 V at a constant current of 1.0 C, (initial) recovery capacity was measured.

After charging was performed under the same charging and discharging conditions, the battery was stored in a thermostat at 60 °C for 4 weeks, and then discharged to a discharge voltage of 3 V at a room temperature of 25 °C. Then, the remaining capacity was measured. Then, recovery capacity was measured after 100 cycles under the same charging and discharging conditions, and the average value thereof was calculated. The results are shown in the item of recovery capacity after high-temperature storage in Tables 6 and 7 below.

[Anode swelling evaluation]

Using a compression-type thickness gauge (Mitutoyo Co.), the pouch cell-type secondary battery was placed between compression plates, and the thickness thereof was measured while the pouch cell was compressed with a weight of 300 g. To exclude the cooling effect, a thickness value (expansion thickness) measured immediately after removal from an oven at 60 °C and a thickness value measured according to the same method after storage for 4 weeks in a constant temperature bath at 60 °C were substituted into Equation 1 below to calculate thickness increase rate (%).

[Equation 1]

$$\text{Thickness increase rate (\%)} = \{(\text{Thickness after storage at high temperature} - \text{Initial thickness}) / \text{Initial thickness}\} \times 100$$

[Evaluation of Coulombic efficiency]

The secondary battery was charged with constant current at 45 °C at a current of 1 C rate until voltage reached 4.20 V (vs. Li), and then cut off at a current of 0.05 C rate while maintaining 4.20 V in a constant voltage mode.

Subsequently, discharge was performed at a constant current of 1 C rate until voltage reached 3.0 V (vs. Li) during discharge.

The above cycle was repeated 300 times, and the charge capacity and the discharge capacity were substituted into Equation 2 to calculate the Coulombic efficiency.

[Equation 2]

Coulomb efficiency (%) = (Discharge capacity at 300 cycles / charge capacity at 300 cycles) × 100

[Table 6]

| Classif ication | First additi ve (wt%) | Seco ndaddi tive (wt% ) | Initi al disch argeresis tance (mQ) | Recov ery capac ity after stora ge at high tempe ratur e (mAh) | Init ial thic knes s (mm) | Expa nsio n thickness (mm) | Thic knes s incr ease rate (%) | Coulo mbeffic iency (%) |
|---|---|---|---|---|---|---|---|---|
| Example 1-1 | Formul a 1a (5) | Form ula 2a (3 ) | 32.68 | 1096 | 3.7 | 4.0 | 8.11 | 99.6 |
| Example 1-2 | Formul a 1a (5) + Formul a 1b (5) | Form ula 2a (3 ) | 38.57 | 1000 | 3.7 | 4.1 | 10.8 1 | 99.4 |
| Example 2-1 | Formul a 1d (10) | Form ula 2a (3 ) | 29.08 | 11.08 | 3.7 | 3.9 | 5.41 | 99.7 |
| Example 2-2 | Formul a 1d (5) + Formul a 1b (5) | Form ula 2a (3 ) | 34.34 | 1056 | 3.8 | 4.0 | 5.26 | 99.6 |
| Example 2-3 | Formul a 1d (5) + Formul a 1a (5) + Formul a 1b (5) | Form ula 2a (3 ) | 36.40 | 1024 | 3.7 | 4.1 | 10.8 1 | 99.5 |
| Example 3-1 | Formul a 1b (5) | Form ula 2a (3 ) | 36.32 | 1032 | 3.8 | 4.0 | 5.26 | 99.5 |
| Example 4-1 | Formul a 1e (15) | Form ula 2a (3 ) | 25.17 | 1123 | 3.8 | 3.9 | 2.63 | 99.8 |
| Example 4-2 | Formul a 1e (10) + Formul a 1f (10) | Form ula 2a (3 ) | 29.64 | 1101 | 3.7 | 3.9 | 5.41 | 99.7 |
| Example 5-1 | Formul a 1h + lithiu m(0.5) | Form ula 2a (3 ) | 28.31 | 1128 | 3.7 | 3.9 | 5.41 | 99.7 |

[Table 7]

| Classif ication | First addit ive (wt%) | Seco nd addi tive (wt% ) | Initi al disch arge resis tance (mQ) | Recov ery capac ity after stora ge at high tempe ratur e (mAh) | Init ial thic kness (mm) | Expa nsio n thickness (mm) | Thic knes s increase rate (%) | Coulo mb efficiency (%) |
|---|---|---|---|---|---|---|---|---|
| Compara tive Example 1-1 | Formu la 1a (5) | - | 41.66 | 991 | 3.8 | 4.3 | 13.1 6 | 99.4 |
| Referen ce Example 1-1 | Formu la 1a (5) + Formu la 1c (10) | Form ula 2a (3 ) | 32.68 | 1096 | 3.7 | 4.0 | 8.11 | 99.6 |
| Compara tive Example 2-1 | Formu la 1d (5) | - | 38.31 | 1007 | 3.7 | 4.2 | 13.5 1 | 99.5 |
| Compara tive Example 3-1 | Formu la 1b (5) | - | 48.40 | 989 | 3.7 | 4.8 | 29.7 3 | 99.2 |
| Referen ce Example 3-1 | Formu la 1b (3) | Form ula 2a (3 ) | 36.32 | 1032 | 3.8 | 4.0 | 5.26 | 99.5 |
| Compara tive Example 4-1 | Formu la 1e (10 ) + Formu la 1g (5) | Form ula 2a (3 ) | 25.17 | 1123 | 3.7 | 3.9 | 5.41 | 99.8 |
| Compara tive Example 4-2 | Formu la 1e (15 ) | - | 34.57 | 1076 | 3.7 | 4.0 | 8.11 | 99.5 |
| Compara tive Example 5-1 | Formu la 1h + lithi um (10 ) | - | 34.80 | 1066 | 3.7 | 4.1 | 10.8 1 | 99.5 |
| Compara tive Example 5-2 | Formu la 1h + sodiu m (0.5 ) | Form ula 2a (3 ) | 30.87 | 1080 | 3.8 | 4.0 | 5.26 | 99.5 |
| Compara tive Example 5-3 | Formu la 1h + lithi um (10 ) + Formu la 1g (5) | Form ula 2a (3 ) | 28.31 | 1128 | 3.7 | 3.9 | 5.41 | 99.7 |

As shown in Tables 6 and 7, in the case of the secondary batteries of Examples 1-1 to 5-1 using the electrolyte solution additives of the present invention, the discharge resistance values thereof were 25.17 to 38.57 mQ. Comparative Examples 1-1 to 5-3 using one type of the first and second additives exhibited high discharge resistance values (25.17

to 48.40 mQ) . Based on these results, it was confirmed that the discharge resistance value was reduced by up to 48 % by using the electrolyte solution additives of the present invention. These results indicate that the output of a battery may be improved by the electrolyte solution additives of the present invention. In addition, as shown in Table 1, the secondary batteries of Examples 1-1 to 5-1 using the electrolyte solution additives of the present invention had a high-temperature recovery capacity of 1,000 to 1,128 mAh. On the other hand, Comparative Examples 1-1 to 5-3 using only one of the first and second additives showed results (989 to 1,128 mAh) similar to those of Examples 1-1 to 5-1.

In addition, as a result of checking anode expansion, in the case of the secondary batteries of Examples 1-1 to 5-1 using the electrolyte solution additives of the present invention, the thickness increase rates thereof were 2.63 to 10.81 %. In contrast, Comparative Examples 1-1 to 5-3 using one type of the first and second additives exhibited 5.26 to 29.73 %, which are up to 27.1 % points lower than Examples of the present invention. These results indicate that, compared to a conventional electrolyte solution additive, use of the electrolyte solution additives of the present invention may improve the capacity retention of the battery while repeating 300 cycles at a high temperature of 60 °C. Accordingly, it can be confirmed that use of the electrolyte solution additives of the present invention may improve high-temperature storage properties and lifespan properties.

In addition, as a result of evaluating coulomb efficiency, in the case of the secondary batteries using the electrolyte solution additives of the present invention, the coulomb efficiency thereof was 99.4 to 99.8 %. In contrast, Comparative Examples 1 and 2 exhibited 99.2 and 99.8 %, which are values up to 0.6 % points lower than Examples of the present invention. These results indicate that, compared to a conventional electrolyte solution additive, use of the electrolyte solution additives of the present invention may improve the capacity retention of the battery while repeating 300 cycles at a high temperature of 60 °C. Accordingly, it can be confirmed that the cycle characteristics and lifespan efficiency of the battery in a high-temperature environment may be improved by using the electrolyte solution additives of the present invention.

Therefore, when the electrolyte solution additives of the present invention and the electrolyte solution including the same are applied to a secondary battery, the discharge resistance, output, recovery capacity, and lifespan efficiency of the secondary battery may be improved. Due to these advantages, the secondary battery according to the present invention is suitable for use as a secondary battery for energy storage systems (ESSs) or a secondary battery for automobiles.

## Claims

1. An electrolyte solution, comprising an organic solvent, a lithium salt, a first additive, and a second additive,

   wherein the first additive comprises a compound represented by Formula 1 below in an amount of 20 % by weight or less based on 100 % by weight of the electrolyte solution, and
   the second additive consists of 3 to 5 atoms, has 2 to 4 atoms having an electronegativity of 3 or more, has at least one double bond, and comprises a compound having an atomic group represented by Formula 2 below in an amount of 0.01 to 10 % by weight based on 100 % by weight of the electrolyte solution.

   [Formula 1]

   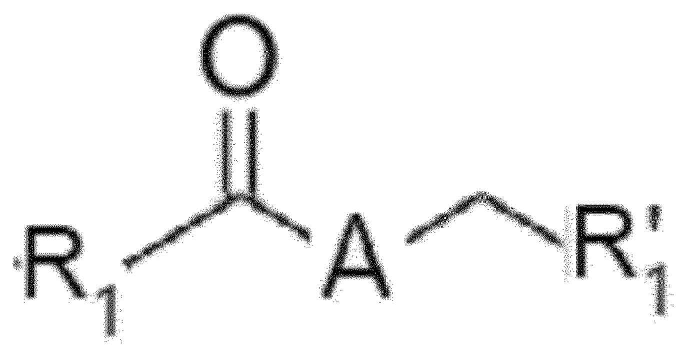

   ,

   wherein A is carbon (C) or oxygen (O); and $R_1$ and $R_1$' are independently a bond or a substituted or unsubstituted alkyl having 1 to 10 carbon atoms and comprise one or more carbon atoms.

[Formula 2]

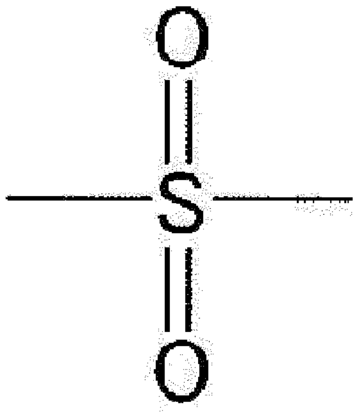

,

wherein a solid line is a bond.

2. The electrolyte solution according to claim 1, wherein, in Formula 1, A is oxygen (O).

3. The electrolyte solution according to claim 1, wherein the first additive is a compound represented by Formula 3 or 4 below.

[Formula 3]

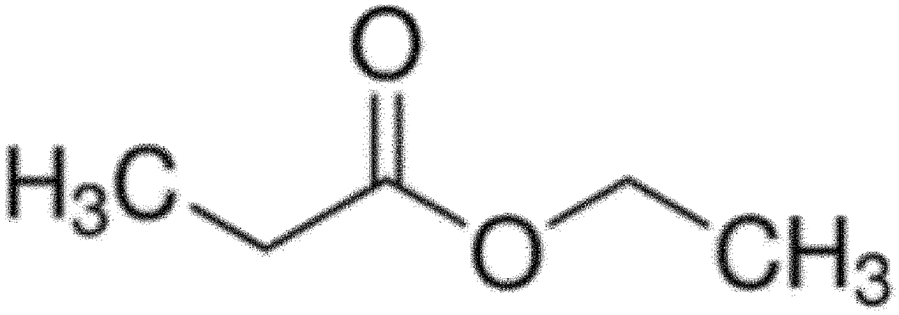

[Formula 4]

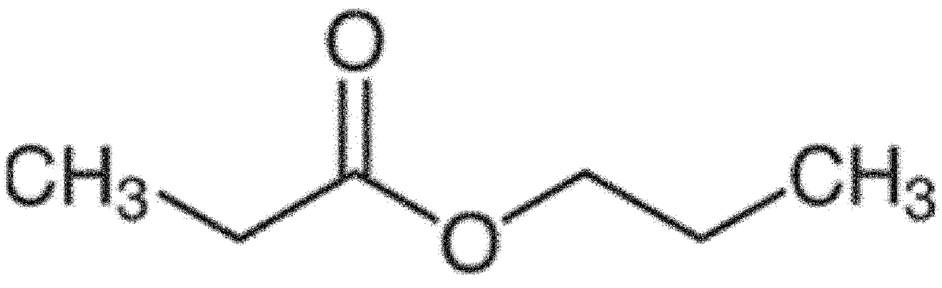

wherein, in Formulas 3 and 4, a solid line is a bond; carbon is located at a point where no separate element is shown and bonds meet; and the number of hydrogens satisfying a valence of the carbon is omitted.

4. The electrolyte solution according to claim 1, wherein the second additive has an atomic group represented by Formula 5 below.

[Formula 5]

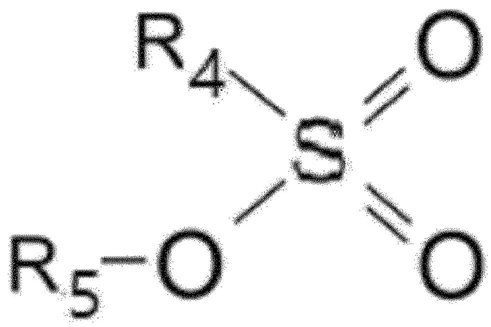

,

wherein $R_4$ is $OR_6$ or $R_6$; $R_5$ is $R_7A$; A is

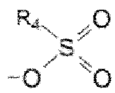

;

and $R_6$ and $R_7$ are independently an alkyl group having 1 to 5 carbon atoms or an aryl group having 6 to 10 carbon atoms.

**5.** The electrolyte solution according to claim 1, wherein the second additive is one or more compound represented by Formula 6 below.

[Formula 6]

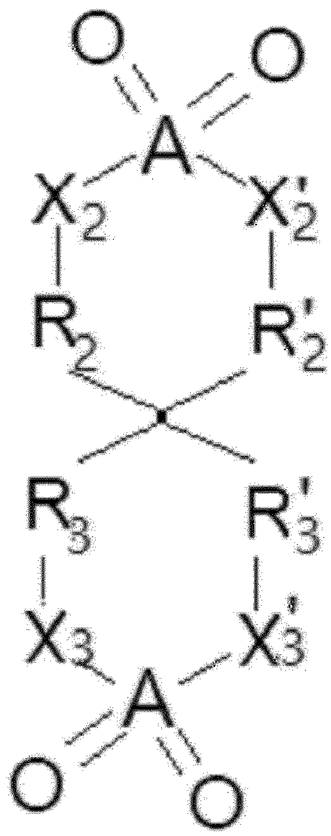

,

wherein A is phosphorus (P), sulfur (S), or nitrogen (N); $X_1$, $X_2$, $X_3$, $X_1'$, $X_2'$, and $X_3'$ are independently a bond or oxygen; n is an integer from 0 to 3; and $R_2$, $R_2$, $R_3$, $R_1'$, $R_2'$, and $R_3'$ are independently a bond or a substituted or unsubstituted alkylene having 1 to 10 carbon atoms and comprise one or more carbon atoms.

6. The electrolyte solution according to claim 5, wherein the second additive is a compound represented by Formula 7 below.

[Formula 7]

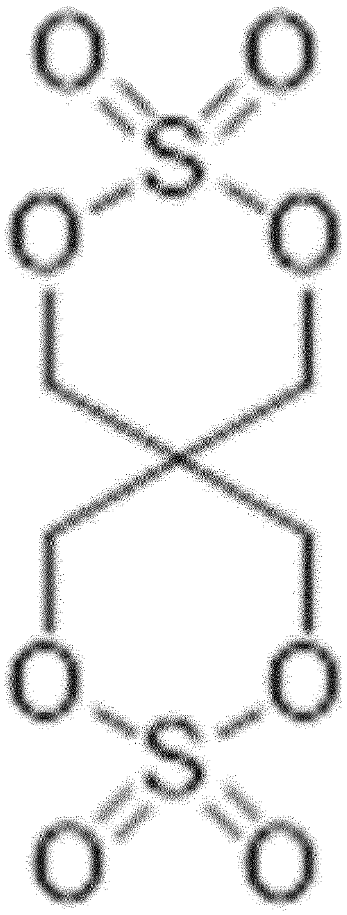

,

wherein a solid line is a bond; carbon is located at a point where no separate element is shown and bonds meet; and the number of hydrogens satisfying a valence of the carbon is omitted.

7. The electrolyte solution according to claim 1, wherein the first and second additives are comprised in a weight ratio of 1:0.1 to 1.7 (first additive:second additive).

8. The electrolyte solution according to claim 1, wherein the lithium salt comprises one or more selected from the group consisting of $LiPF_6$, $LiClO_4$, LiTFSI, $LiAsF_6$, $LiBF_4$, $LiBF_6$, $LiSbF_6$, $LiAl0_4$, $LiAlCl_4$, LiBOB, $LiB_{10}Cl_{10}$, $LiAlCl_4$, $LiAlO_4$, $LiClO_4$, $LiCF_3SO_3$, $LiCH_3CO_2$, $LiCF_3CO_2$, $LiAsF_6$, $LiSbF_6$, $LiCH_3SO_3$, LiBeTI, $LiC_4F_9SO_3$, LiN $(C_2F_5SO_3)_2$, LiN $(C_2F_5SO_2)_2$, LiN $(CF_3SO_2)_2$, $LiN(CaF_{2a+1}SO_2)(C_bF_{2b+1}SO_2)$ (a and b are natural numbers), LiCl, LiI, LiBr, and $LiB(C_2O_4)_2$.

9. The electrolyte solution according to claim 1, wherein the organic solvent comprises two or more selected from the group consisting of ethylene carbonate (EC), propylene carbonate (PC), butylene carbonate (BC), vinylene carbonate (VC), dimethyl carbonate (DMC), diethyl carbonate (DEC), dipropyl carbonate (DPC), ethyl methyl carbonate (EMC), methyl ethyl carbonate (MEC), fluoroethylene carbonate (FEC), methyl propyl carbonate (MPC), ethyl propyl carbonate (EPC), 1,2-butylene carbonate, 2,3-butylene carbonate, 1,2-pentylene carbonate, 2,3-butylene carbonate,

methyl acetate, ethyl acetate, propyl acetate, methyl propionate, butyl propionate, γ-butyrolactone, γ-valerolactone, γ-caprolactone, σ-valerolactone, and ε-caprolactone.

**10.** The electrolyte solution according to claim 1, wherein, based on 100 % by weight in total of the electrolyte solution, the electrolyte solution comprises one or more third additives selected from the group consisting of boron compounds, phosphorus compounds, sulfur compounds, and nitrogenous compounds in an amount of 10 % by weight or less.

**11.** Electrolyte solution additives, comprising a compound represented by Formula 1 below; and a compound represented by Formula 4 below.

[Formula 1]

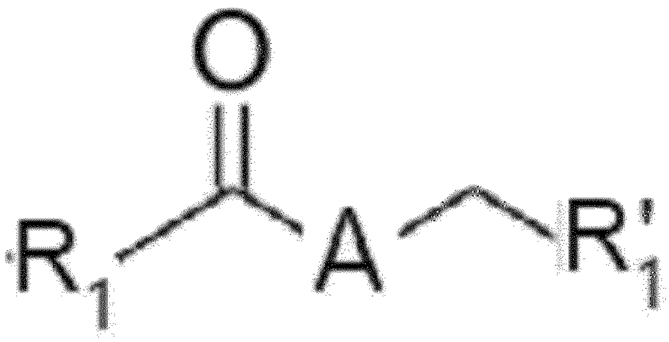

,

wherein A is carbon (C) or oxygen (O); and $R_1$ and $R_1$' are independently a bond or a substituted or unsubstituted alkyl having 1 to 10 carbon atoms and comprise one or more carbon atoms.

[Formula 5]

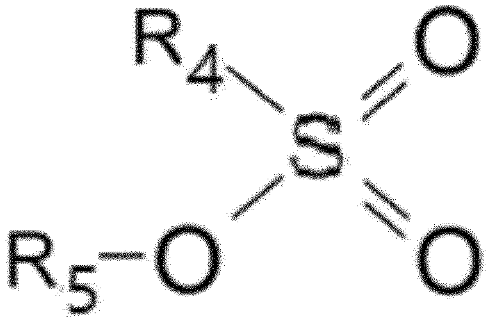

,

wherein $R_4$ is $OR_6$ or $R_6$; $R_5$ is $R_7A$; A is

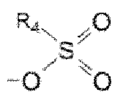

;

and $R_6$ and $R_7$ are independently an alkyl group having 1 to 5 carbon atoms or an aryl group having 6 to 10 carbon atoms.

**12.** The electrolyte solution additives according to claim 11, wherein the compound represented by Formula 1 and the compound represented by Formula 5 are comprised in a weight ratio of 1:0.1 to 1.7 (first additive:second additive).

**13.** A lithium secondary battery, comprising an anode, a cathode, a separator disposed between the anode and the

cathode, and an electrolyte solution, wherein the electrolyte solution is the electrolyte solution according to any one of claims 1 to 10.

**14.** The lithium secondary battery according to claim 11, wherein the lithium secondary battery has a discharge resistance value of 40 mQ or less at 60 °C.

**15.** The lithium secondary battery according to claim 11, wherein the lithium secondary battery has a recovery capacity of 1,000 mAh or more at 60 °C.

**16.** The lithium secondary battery according to claim 11, wherein the lithium secondary battery has a thickness increase rate of 10.81 % or less as measured at 60 °C and calculated by Equation 1 below.

[Equation 1]

Thickness increase rate (%) = {(Thickness after storage at high temperature - Initial thickness) / Initial thickness} × 100

**17.** The lithium secondary battery according to claim 11, wherein the lithium secondary battery has a coulomb efficiency of 99.4 % or more as calculated by Equation 2 below after 300 cycles.

[Equation 2]

Coulomb efficiency (%) = (Discharge capacity at 300 cycles / charge capacity at 300 cycles) × 100

**18.** The lithium secondary battery according to claim 11, wherein the lithium secondary battery is a battery for energy storage systems (ESSs) or a battery for automobiles.

INTERNATIONAL SEARCH REPORT

International application No.
**PCT/KR2022/001172**

**A. CLASSIFICATION OF SUBJECT MATTER**

**H01M 10/0567**(2010.01)i; **H01M 10/052**(2010.01)i

According to International Patent Classification (IPC) or to both national classification and IPC

**B. FIELDS SEARCHED**

Minimum documentation searched (classification system followed by classification symbols)

H01M 10/0567(2010.01); H01M 10/052(2010.01); H01M 10/0525(2010.01); H01M 4/48(2010.01); H01M 4/58(2010.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, CAplus) & keywords: 첨가제(additive), 전해액(electrolyte), 리튬 이차전지 (lithium secondary battery)

**C. DOCUMENTS CONSIDERED TO BE RELEVANT**

| Category* | Citation of document, with indication, where appropriate, of the relevant passages | Relevant to claim No. |
|---|---|---|
| Y | KR 10-2019-0092879 A (PANAX ETEC CO., LTD.) 08 August 2019 (2019-08-08)<br>See claims 1, 4, 5, 8 and 10; paragraphs [0002] and [0048]; example 1; and table 1. | 1-10,13-18 |
| Y | KR 10-2019-0115949 A (SAMSUNG SDI CO., LTD.) 14 October 2019 (2019-10-14)<br>See claims 1, 6 and 7; and paragraphs [0042] and [0044]. | 1-18 |
| Y | KR 10-2007-0095222 A (HITACHI MAXELL, LTD.) 28 September 2007 (2007-09-28)<br>See abstract; and paragraphs [0129] and [0130]. | 11,12 |
| Y | KR 10-2017-0094966 A (SAMSUNG SDI CO., LTD.) 22 August 2017 (2017-08-22)<br>See claims 1, 10-13 and 17; paragraphs [0002] and [0086]; and example 1. | 1-10,13-18 |
| A | US 2020-0313237 A1 (SHENZHEN CAPCHEM TECHNOLOGY CO., LTD.) 01 October 2020 (2020-10-01)<br>See entire document. | 1-18 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **09 May 2022** | **09 May 2022** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2019)

**INTERNATIONAL SEARCH REPORT**
**Information on patent family members**

International application No.
**PCT/KR2022/001172**

| Patent document cited in search report | Publication date (day/month/year) | Patent family member(s) | Publication date (day/month/year) |
|---|---|---|---|
| KR 10-2019-0092879 A | 08 August 2019 | KR 10-2023677 B1 | 23 September 2019 |
| KR 10-2019-0115949 A | 14 October 2019 | KR 10-2288619 B1 | 10 August 2021 |
| KR 10-2007-0095222 A | 28 September 2007 | CN 101188282 A | 28 May 2008 |
| | | CN 101188282 B | 08 September 2010 |
| | | JP 2007-287658 A | 01 November 2007 |
| | | JP 2007-294396 A | 08 November 2007 |
| | | JP 2007-294397 A | 08 November 2007 |
| | | JP 5117729 B2 | 16 January 2013 |
| | | JP 5117730 B2 | 16 January 2013 |
| | | JP 5213151 B2 | 19 June 2013 |
| | | KR 10-1370675 B1 | 04 March 2014 |
| | | US 2007-0224504 A1 | 27 September 2007 |
| | | US 9077035 B2 | 07 July 2015 |
| KR 10-2017-0094966 A | 22 August 2017 | CN 107086324 A | 22 August 2017 |
| | | CN 107086324 B | 23 July 2021 |
| | | CN 110931853 A | 27 March 2020 |
| | | CN 110931854 A | 27 March 2020 |
| | | CN 110931855 A | 27 March 2020 |
| | | CN 110931856 A | 27 March 2020 |
| | | CN 110931857 A | 27 March 2020 |
| | | CN 110931858 A | 27 March 2020 |
| | | CN 110931859 A | 27 March 2020 |
| | | EP 3205655 A1 | 16 August 2017 |
| | | EP 3205655 B1 | 20 June 2018 |
| | | KR 10-2020-0033198 A | 27 March 2020 |
| | | KR 10-2020-0033199 A | 27 March 2020 |
| | | KR 10-2020-0033200 A | 27 March 2020 |
| | | KR 10-2020-0033201 A | 27 March 2020 |
| | | KR 10-2020-0033202 A | 27 March 2020 |
| | | KR 10-2020-0033203 A | 27 March 2020 |
| | | KR 10-2020-0033204 A | 27 March 2020 |
| | | KR 10-2152365 B1 | 04 September 2020 |
| | | KR 10-2332336 B1 | 29 November 2021 |
| | | US 1114694 B2 | 07 September 2021 |
| | | US 1127978 B2 | 21 September 2021 |
| | | US 1145900 B2 | 12 October 2021 |
| | | US 1223044 B2 | 11 January 2022 |
| | | US 1251432 B2 | 15 February 2022 |
| | | US 1264644 B2 | 01 March 2022 |
| | | US 1264645 B2 | 01 March 2022 |
| | | US 2017-0237126 A1 | 17 August 2017 |
| | | US 2019-0020064 A1 | 17 January 2019 |
| | | US 2019-0020065 A1 | 17 January 2019 |
| | | US 2019-0020066 A1 | 17 January 2019 |
| | | US 2019-0020067 A1 | 17 January 2019 |
| | | US 2019-0020068 A1 | 17 January 2019 |
| | | US 2019-0020070 A1 | 17 January 2019 |
| | | US 2019-0020071 A1 | 17 January 2019 |
| US 2020-0313237 A1 | 01 October 2020 | CN 109950621 A | 28 June 2019 |
| | | EP 3731325 A1 | 28 October 2020 |

Form PCT/ISA/210 (patent family annex) (July 2019)

**INTERNATIONAL SEARCH REPORT**
**Information on patent family members**

International application No.
**PCT/KR2022/001172**

| Patent document cited in search report | Publication date (day/month/year) | Patent family member(s) | Publication date (day/month/year) |
|---|---|---|---|
| | | EP 3731325 B1 | 29 December 2021 |
| | | WO 2019-119765 A1 | 27 June 2019 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1295395 B1


### Non-patent literature cited in the description


- Battery test manual for plug-in hybrid electric vehicles. Idaho National Laboratory for the U.S. Department of Energy, 2010